(19) Europäisches Patentamt
European Patent Office
Office européen des brevets

(11) Publication number: **0 127 848 A2**

(12) **EUROPEAN PATENT APPLICATION**

(21) Application number: 84105921.5

(22) Date of filing: 24.05.84

(51) Int. Cl.³: **C 07 C 149/00**
C 07 C 147/14, C 07 C 147/00
A 61 K 31/19

(30) Priority: 02.06.83 US 500606

(43) Date of publication of application: 12.12.84 Bulletin 84/50

(84) Designated Contracting States: CH DE FR GB IT LI NL

(71) Applicant: MERCK & CO. INC.
126, East Lincoln Avenue P.O. Box 2000
Rahway New Jersey 07065(US)

(72) Inventor: Prugh, John D.
16 Far View Road
Chalfont Pennsylvania 18914(US)

(74) Representative: Abitz, Walter, Dr.-Ing. et al,
Abitz, Morf, Gritschneder, Freiherr von Wittgenstein
Postfach 86 01 09
D-8000 München 86(DE)

(54) **5-Thiaalkanoic acid derivatives having antihypercholesterolemic activity.**

(57) Derivatives of 3-hydroxy-5-thia-ω-aryl-alkanoic acids are disclosed having the structural formula:

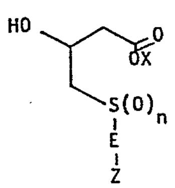

wherein Z is:

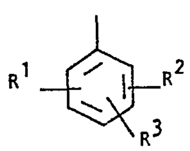

or

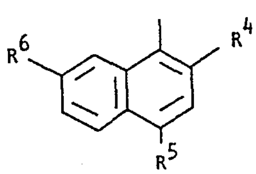

n is 0, 1 or 2;

E is $-CH_2-$, $-CH_2-CH_2-$, $-CH_2-CH_2-CH_2-$, $-CH=CH-CH_2-$; or $-CH_2-CH=CH-$;

$R_1$, $R_2$ and $R_3$ are, e.g., hydrogen, chloro, bromo, fluoro, $C_{1-4}$alkyl, phenyl, substituted phenyl or $OR_7$ in which $R_7$ is, e.g., hydrogen,
$C_{2-8}$alkanoyl, benzoyl, phenyl,
substituted phenyl, $C_{1-9}$alkyl,
cinnamyl, $C_{1-4}$haloalkyl, allyl,
cycloalkyl-$C_{1-3}$-alkyl, adamantyl-$C_{1-3}$-alkyl,
or phenyl $C_{1-3}$ alkyl;

$R^4$, $R^5$ and $R^6$ are gydrogen, chloro, bromo, fluoro or $C_{1-3}$ alkyl; and

X is, e.g., hydrogen, $C_{1-3}$ alkyl, a cation derived from an alkali metal, or is ammonium.

Those compounds have antihypercholesterolemic activity by virtue of their ability to inhibit 3-hydroxy-3-methylglutaryl-coenzyme A (HMG-CoA) reductase and antifungal acticity.

EP 0 127 848 A2

Croydon Printing Company Ltd

TITLE OF THE INVENTION

5-THIAALKANOIC ACID DERIVATIVES HAVING ANTIHYPERCHOLESTEROLEMIC ACTIVITY

SUMMARY OF THE INVENTION

This invention is concerned with novel compounds of the general structural formula:

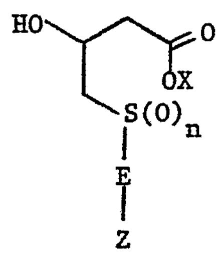

wherein Z is a substituted or unsubstituted phenyl or naphthyl group.

It is also concerned with processes for preparing the novel compounds; antihypercholesterolemic pharmaceutical formulations comprising one of the novel compounds as active ingredient; a method of

treating atherosclerosis, familial hypercholesterolemia, hyperlipemia and like diseases with a novel compound or pharmaceutical formulation thereof; antifungal formulations comprising one of the novel compounds as active ingredient; and a method of treating fungal infestations with a novel compound or formulation thereof.

## BACKGROUND OF THE INVENTION

Compounds of the structural formula:

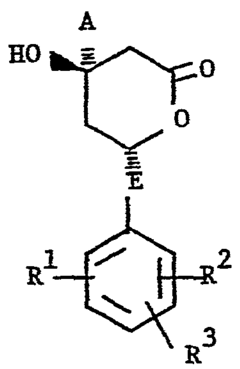

II(a)

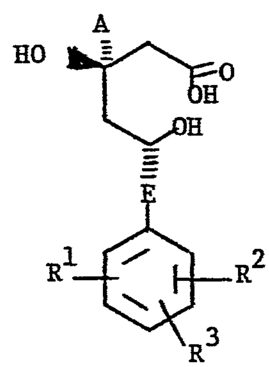

II(b)

wherein A is hydrogen or methyl and the other variable substituents are substantially similar to those of the novel compounds of this invention, are known from U.S. Patent 4,375,475 and known to have utility as antihypercholesterolemic and antifungal agents. These compounds are very active and useful compounds, however, the more active analogs are quite difficult to synthesize in isomerically pure form, especially on a large scale.

Now with the present invention there are provided novel compounds of comparable activity and utility and which are more easily synthesized, especially in those compounds wherein the thia

heteroatom is in unoxidized form as the stereochemical complicity is lacking.

DETAILED DESCRIPTION OF THE INVENTION

The novel compounds of the present invention have structural formula I:

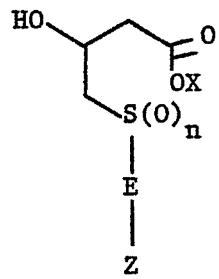

wherein Z is:

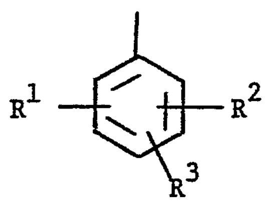

or

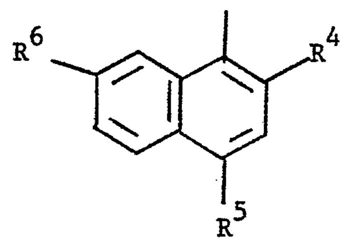

n is 0, 1 or 2;

E is $-CH_2-$, $-CH_2-CH_2-$, $-CH_2-CH_2-CH_2-$, $-CH=CH-CH_2-$, or $-CH_2-CH=CH-$;

$R_1$, $R_2$ and $R_3$ are each selected from:

1) hydrogen,
2) halogen, such as chloro, bromo or fluoro,
3) $C_{1-4}$alkyl,
4) $C_{1-4}$haloalkyl, wherein halo is chloro, bromo or fluoro,

5) phenyl,

6) phenyl substituted by one or more of:
   a) halogen,
   b) $C_{1-4}$ alkoxy,
   c) $C_{2-8}$ alkanoyloxy,
   d) $C_{1-4}$ alkyl, or
   e) $C_{1-4}$ haloalkyl, and

7) $OR_7$ in which $R_7$ is
   a) hydrogen,
   b) $C_{2-8}$ alkanoyl,
   c) benzoyl,
   d) phenyl,
   e) phenyl substituted with one or more of
      i) halogen,
      ii) $C_{1-4}$ alkoxy,
      iii) $C_{2-8}$ alkanoyloxy,
      iv) $C_{1-4}$ alkyl, or
      v) $C_{1-4}$ haloalkyl,
   f) $C_{1-9}$ alkyl,
   g) cinnamyl,
   h) $C_{1-4}$ haloalkyl,
   i) allyl,
   j) cycloalkyl-$C_{1-3}$-alkyl,
   k) adamantyl-$C_{1-3}$-alkyl,
   l) phenyl $C_{1-3}$ alkyl,
   m) phenyl $C_{1-3}$-alkyl, wherein the phenyl is substituted with one or more of:
      i) halogen,
      ii) $C_{1-4}$ alkoxy,
      iii) $C_{2-8}$ alkanoyloxy,

iv) $C_{1-4}$ alkyl, or

v) $C_{1-4}$ haloalkyl; and

$R^4$, $R^5$ and $R^6$ are each selected from:

1) hydrogen,

2) halo, such as chloro, bromo or fluoro, and

3) $C_{1-3}$ alkyl; and

X is 1) hydrogen,

2) $R^8$ wherein $R^8$ is $C_{1-3}$ alkyl,

3) M wherein M is a cation derived from an alkali metal, such as sodium or potassium, or is ammonium of formula $^+NR^9$, $R^{10}$, $R^{11}$, $R^{12}$ wherein $R^9$, $R^{10}$, $R^{11}$ and $R^{12}$ are independently $C_{1-3}$ alkyl, or two of $R^9$, $R^{10}$, $R^{11}$ and $R^{12}$ are joined together to form a 5 or 6-membered heterocycle such as pyrrolidino or piperidino with the nitrogen to which they are attached,

4) M' wherein M' is a cation derived from protonation of an amine of formula $NR^{13}R^{14}R^{15}$ wherein $R^{13}$,$R^{14}$ and $R^{15}$ are independently hydrogen or $C_{1-3}$ alkyl, or two of $R^{13}$, $R^{14}$ and $R^{15}$ are joined to form a 5 or 6-membered heterocycle such as pyrrolidino or piperidino, with the nitrogen to which they are attached, or

5) $R^{16}$ wherein $R^{16}$ is

a) $C_{1-5}$ alkyl, or

b) 2,3-dihydroxypropyl.

A preferred embodiment of this invention is the compounds of general formula I wherein

Z is: 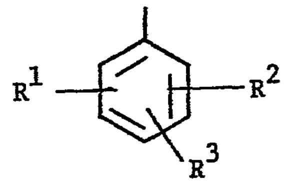

n = 0, 1 or 2;

E is $-CH_2CH_2-$;

$R_1$, $R_2$ and $R_3$ are each selected from:

1) halogen,
2) $C_{1-4}$ alkyl,
3) $C_{1-4}$ haloalkyl,
4) phenyl substituted by one or more of
   a) halo,
   b) $C_{1-4}$ alkyl,
   c) $C_{1-4}$ alkoxy, and
5) $OR_7$ in which $R_7$ is
   a) phenyl,
   b) phenyl substituted by one or more of
      i) halogen, or
      ii) $C_{1-4}$ alkyl;
   c) phenyl-$C_{1-3}$ alkyl, or
   d) phenyl-$C_{1-3}$-alkyl substituted with one or more of
      i) halogen and
      ii) $C_{1-4}$ alkyl; and

X is
1) hydrogen,
2) $R^8$,
3) M, or
4) M'

A more preferred embodiment of the present invention comprises the compounds of general formula I wherein

Z is 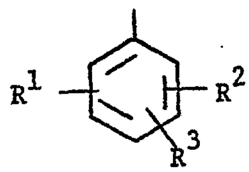

n is 0 or 1;

E is $-CH_2-CH_2-$;

$R_1$ is situated in the 6-position and is a substituted phenyl wherein there are 1 or 2 substituents and they are independently selected from chloro, fluoro, methyl and methoxy; and

$R_2$ and $R_3$ are halo, especially chloro, or $C_{1-3}$alkyl, especially methyl, in the 2 and 4 positions; and

X is hydrogen, a sodium cation, or $C_{1-3}$ alkyl.

Most preferred are the compounds of structural formula:

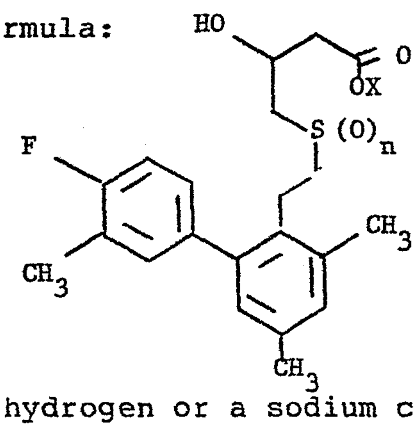

wherein X is hydrogen or a sodium cation, and n = 0 or 1.

In the novel compounds of this invention, wherein n is 1, there are two asymmetric centers, the sulfur and carbon 3 of the thiaalkanoic acid. Silica gel chromatography under conditions described below serves to separate the esters of these alkanoic acids into racemic diastereomeric pairs both of which

demonstrate activity as inhibitors of HMG-CoA reductase. The optical antipodes of each diastereomeric racemate can be separated by known resolution procedures.

The novel processes of this invention for synthesis of the novel compounds are shown in the following Flow Sheets:

FLOW SHEET A

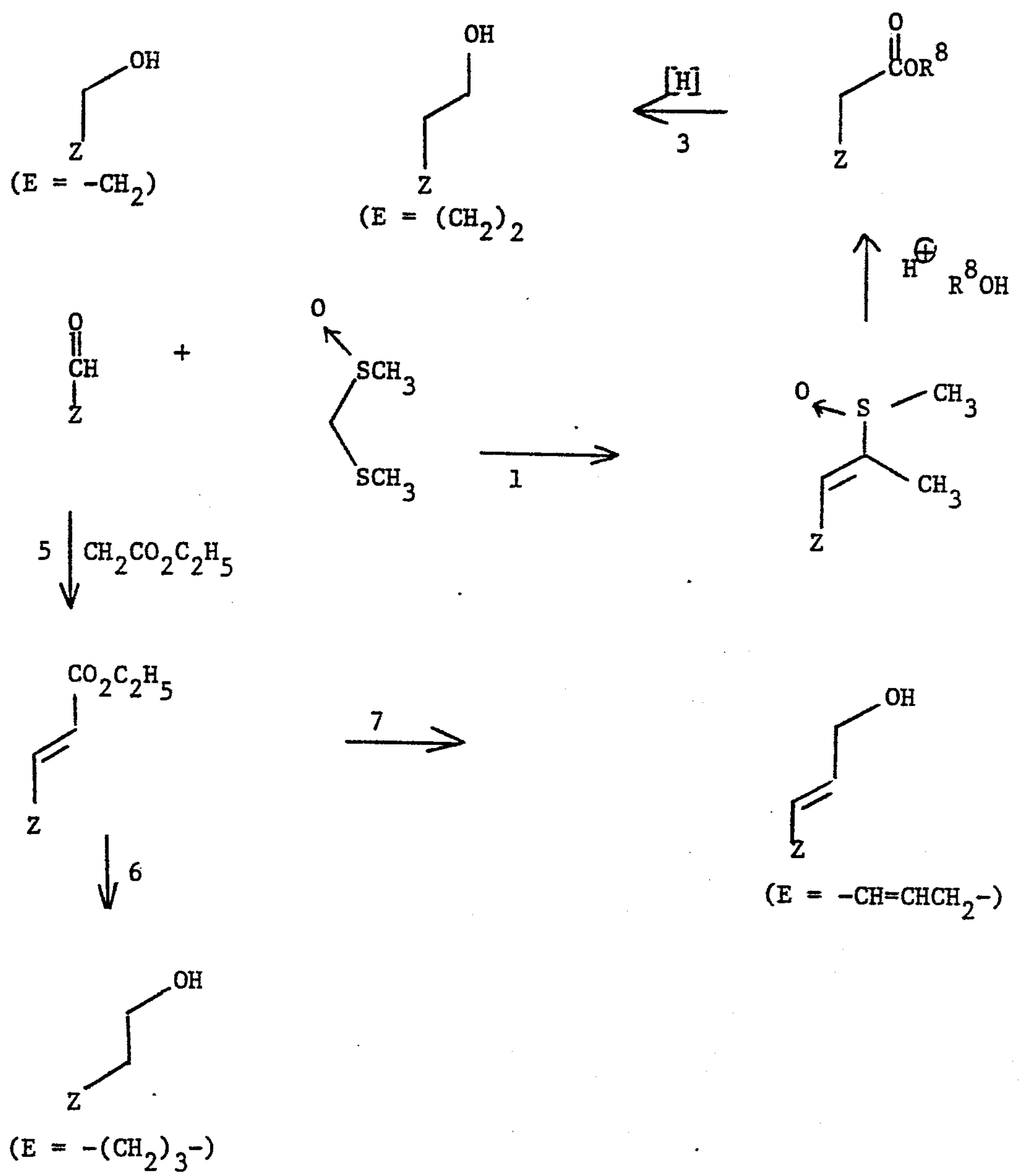

FLOW SHEET B

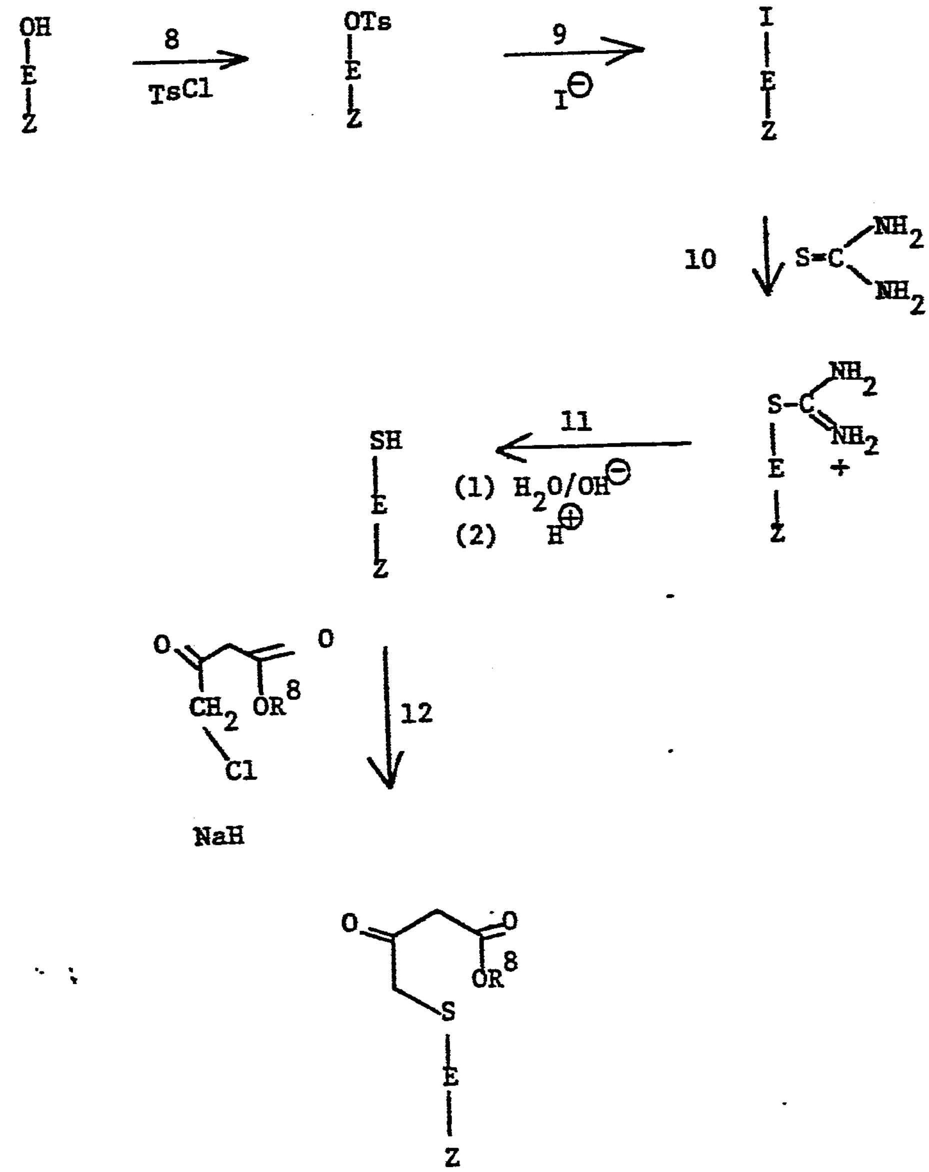

FLOW SHEET C

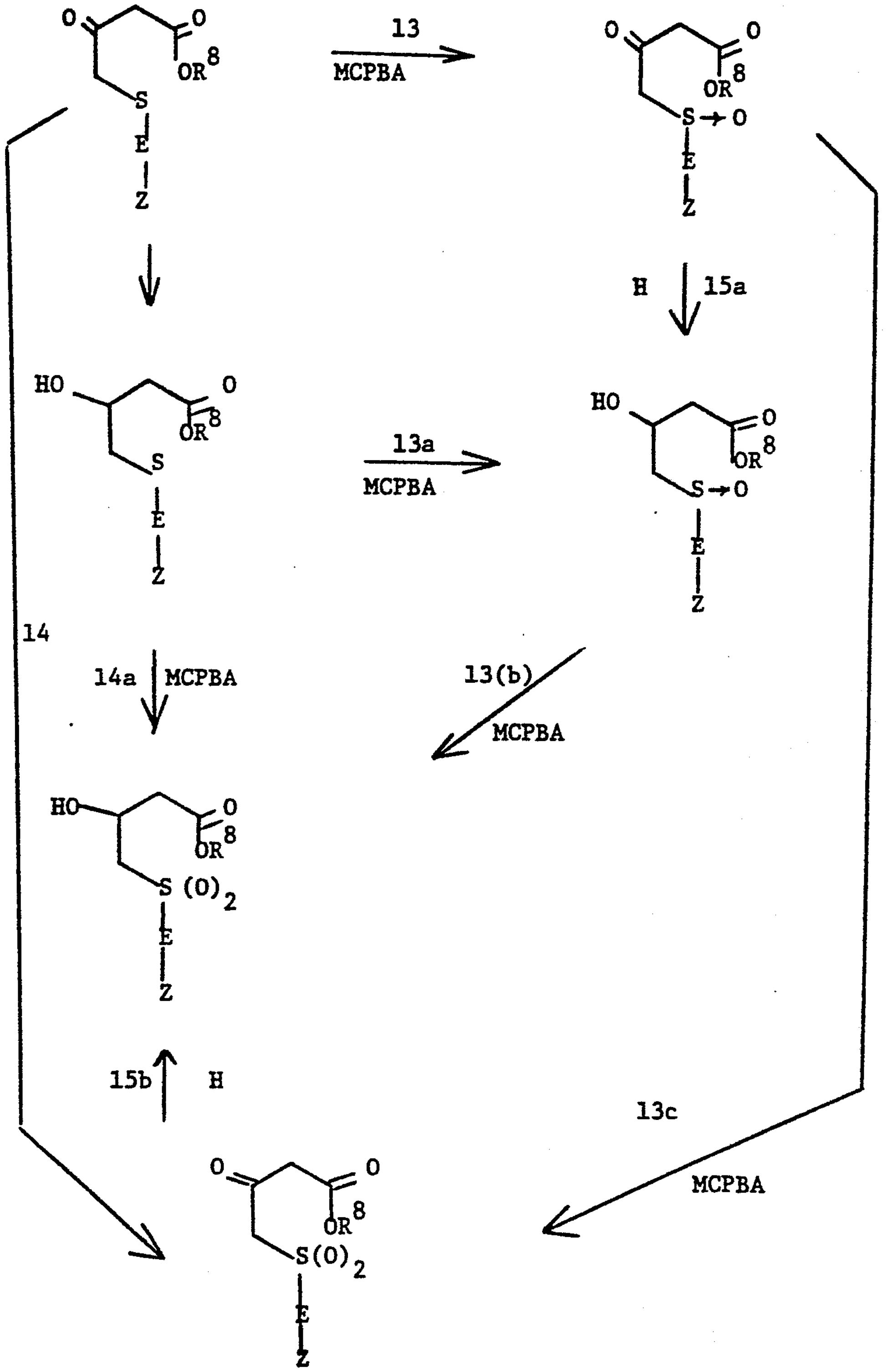

FLOW SHEET D

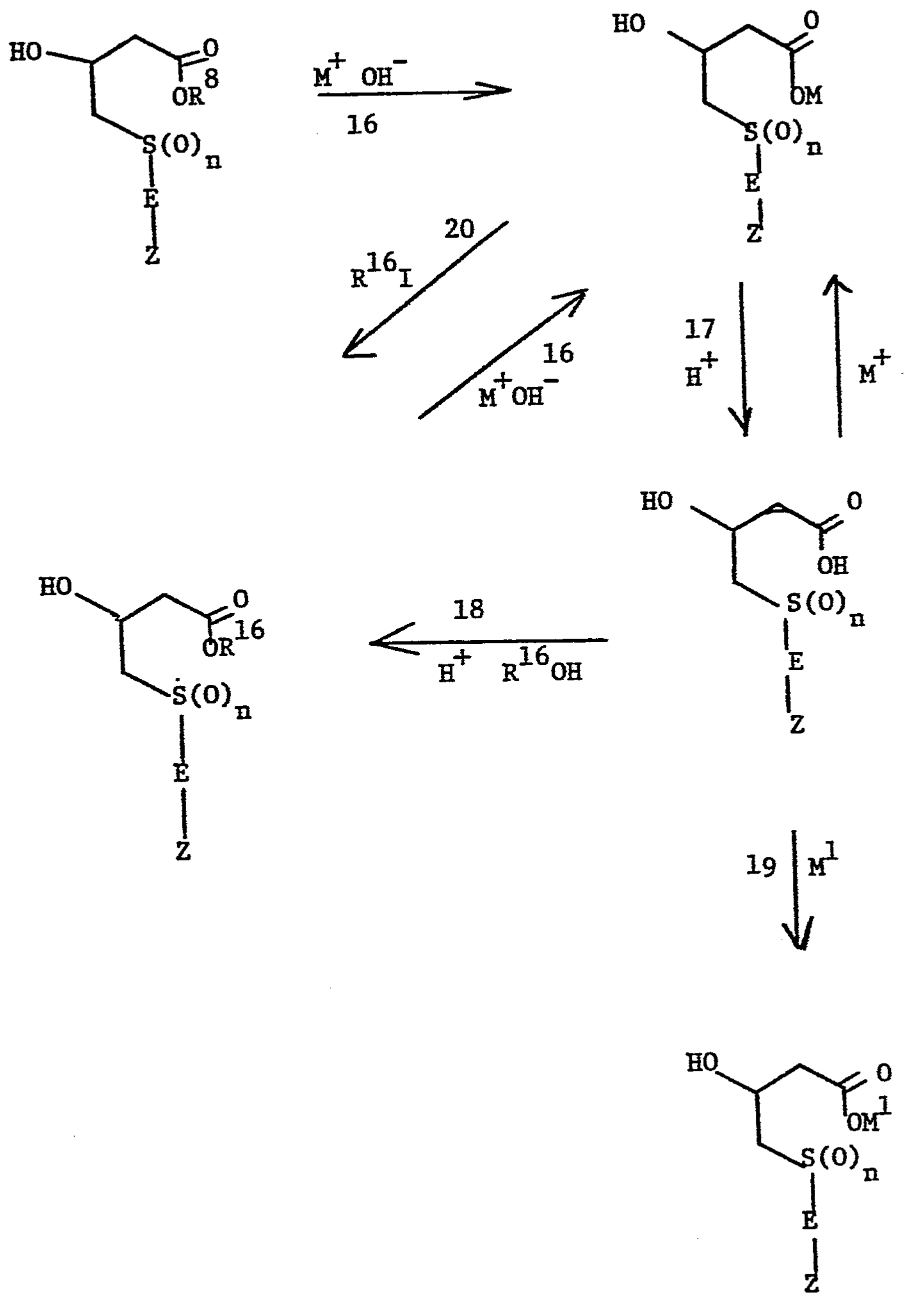

Reactions, Reagents and Conditions

1. A mixture of the appropriately substituted aldehyde and methyl methylsulfinylmethyl sulfide in an ethereal solvent such as tetrahydrofuran, diethyl ether, tetrahydropyran, 1,2-dimethoxyethane or the like is treated with Triton B at about 40 to 100°C for 3 to 10 days.

2. The product of Reaction 1 is treated with a strong inorganic acid in a $C_{1-3}$ alkanol preferably dilute ethanolic hydrogen chloride at about 50 to 100°C, usually reflux temperature, for about 2 to 8 hours.

3. The reduction of the product of Reaction 2 is accomplished with a complex metal hydride such as lithium aluminum hydride, Redal or diisobutyl-aluminum hydride, in an ethereal solvent such as tetrahydrofuran, tetrahydropyran, diethyl ether, 1,2-dimethoxyethane or the like at or near room temperature for about 0.5 to 3 hours.

4. A suspension of the aldenyde in a lower alkanol, such as methanol, ethanol or propanol at about -10 to about +10°C is treated with a complex metal hydride such as sodium borohydride, lithium aluminum hydride or sodium bis(2-methoxyethoxy)-aluminum hydride in benzene with stirring for about 0.5 to 4 hours at ambient temperature.

5. The aldehyde in ethyl acetate solution is added slowly to an ethyl acetate solution of a strong base such as sodium hydride, lithium hydride, or potassium t-butoxide at about -25 to +10°C then aged to ambient temperature for about 8 to 24 hours.

6. The ester product of reaction 5 in ether is treated with a complex metal hydride such as

lithium aluminum hydride, di(isobutyl)aluminum hydride or Redal in ether at about -10 to +10°C then aged at ambient temperature for about 8 to 24 hours.

7. The ester product of reaction 5 in ether is treated with $LiAlH_3OC_2H_5$ ($LiAlH_4+C_2H_5OH$) at about -10 to +10°C for about 30 minutes to 4 hours.

8. The product of Reaction 3, 4, 6 or 7 is treated with a sulfonyl chloride such as p-toluenesulfonyl chloride, benzenesulfonyl chloride, methanesulfonyl chloride, or the like in the presence of at least one equivalent of an organic base such as pyridine, trimethylamine, triethylamine, 4-dimethylaminopyridine or the like in an inert solvent such as benzene, chlorobenzene, chloroform, methylenechloride, or toluene, for about 1 to 5 hours at about 15 to 25°C. Alternatively, the organic base, such as pyridine can also act as the exclusive solvent or in admixture with one of the other recited solvents.

9. The product of Reaction 8 is treated with an alkali metal iodide, preferably sodium iodide in a polar solvent such as acetone, or methyl ethyl ketone at about 30-75°C for about 24 to 96 hours in an inert atmosphere, protected from the light.

10. The alkyl iodide from Reaction 9 is treated with thiourea in a $C_{1-3}$ alkanol, preferably ethanol at about 50 to 100°C, usually reflux temperature in an inert atmosphere for about 3 to 10 days.

11. The isothiuronium iodide from Reaction 10 in a $C_{1-3}$ alkanol, preferably ethanol, is treated with aqueous alkali, especially an alkali metal hydroxide, preferably sodium hydroxide at about 60°C to reflux temperature for about 1 to 6 hours

followed by cooling and acidification with a strong inorganic acid in amount substantially equivalent to the alkali used.

12. The thiol from Reaction 11 is condensed with a $C_{1-3}$ alkyl 4-chloroacetoacetate, preferably methyl 4-chloroacetoacetate, in the presence of a strong base such as sodium hydride, sodium ethoxide, or potassium carbonate in an aprotic solvent such as N,N-dimethylformamide, N-methylpyrrolidone or hexamethylphosphoramide under an inert atmosphere at about -10°C to +10°C followed by spontaneous warming to ambient temperature over a period of about 1 to 5 hours.

13. A sulfide is converted to a sulfoxide or a sulfoxide to the corresponding sulfone by oxidation with approximately 1 mole of m-chloroperbenzoic acid, or peracetic acid in a chlorinated hydrocarbon solvent such as methylene chloride, chloroform, 1,2-dichlorethane or the like at about -80 to -60°C initially, followed by spontaneous warming to about 15-25°C over about 15 minutes to about 90 minutes.

Alternatively the oxidations can be conducted with one mole of $(n\text{-}Bu)_4N^{+}IO_4^{-}$ in refluxing choroform.

14. A sulfide is converted to a sulfone by the procedure described in Reaction 13 but employing 2 moles of oxidizing agent at room temperature.

15. The 3-oxo function is reduced to a secondary alcohol by treatment with a complex metal hydride such as sodium borohydride, in a $C_{1-3}$ alkanol, preferably methanol at about -5 to +5°C for about 5 to 60 minutes.

16. The esters of Structure I ($X = R^8$ or $R^{16}$) are saponified to form the corresponding salts, (X=M) by treatment with aqueous alkali, especially an alkali metal hydroxide, preferably sodium hydroxide, or potassium hydroxide, or an ammonium hydroxide of formula $H\overset{-}{O}\overset{+}{N}R^{9}R^{10}R^{11}R^{12}$.

17. The free acids of Structure I, (X = H) are formed by treating the salts (X = M or M') with one equivalent of a mineral acid.

18. The free acids from Reaction 13 are converted to esters ($X = R^{16}$) by treatment with an alcohol of formula $R^{16}OH$ in the presence of a catalytic quantity of hydrogen ion at about 50 to about 100°C for about 3 to 6 hours.

19. Amine salts (X = M') are formed by treating the acids (X = H) with at least one equivalent of an amine of formula $NR^{13}R^{14}R^{15}$.

20. Salts (X = M) are converted to esters ($X = R^{16}$) by treatment with an alkyl halide of formula $R^{16}$ halide, preferably $R^{16}I$ in an aprotic solvent such as N,N-dimethylformamide, N-methylpyrrolidone or hexamethylphosphoramide at about 25 to 100°C for about 18 to 36 hours.

The novel pharmaceutical composition of this invention comprises at least one of the compounds of formula I in association with a pharmaceutical vehicle or diluent. The pharmaceutical composition can be formulated in a classical manner utilizing solid or liquid vehicles or diluents and pharmaceutical additives of a type appropriate to the mode of desired administration. The compounds can be

administered by an oral route, for example, in the form of tablets, capsules, granules or powders, or they can be administered by a parenteral route in the form of injectable preparations.

A typical capsule for oral administration contains active ingredient (25 mg), lactose (75 mg) and magnesium stearate (15 mg). The mixture is passed through a 60 mesh sieve and packed into a No. 1 gelatin capsule.

A typical injectable preparation is produced by asceptically placing 25 mg of a water soluble salt of sterile active ingredient into a vial, asceptically freeze-drying and sealing. For use, the contents of the vial are mixed with 2 ml of physiological saline, to produce an injectable preparation.

The novel method of treating atherosclerosis, familial hypercholesterolemia, or hyperlipemia of this invention comprises administration of an effective antihypercholesterolemic amount of a compound of Formula I to a patient in need of such treatment.

The dose to be administered depends on the unitary dose, the symptoms, and the age and the body weight of the patient. A dose for adults is preferably between 20 and 2,000 mg per day, which can be administered in a single dose or in the form of individual doses from 1-4 times per day.

The compounds of this invention also have useful antifungal activities. For example, they may be used to control strains of *Penicillium sp.*, *Aspergillus niger*, *Cladosporium sp.*, *Cochliobolus miyabeorus* and *Hilminthosporium cynodnotis*. For

those utilities they are admixed with suitable formulating agents, powders, emulsifying agents or solvents such as aqueous ethanol and sprayed or dusted on the plants to be protected.

This invention can be illustrated by the following examples.

EXAMPLE 1

7-[4'-fluoro-3,3',5-trimethyl-(1,1'-biphenyl)-2-yl]-3-hydroxy-5-thiaheptanoic acid

Step A: Preparation of Ethyl [4'-fluoro-3,3',5-trimethyl-(1,1'-biphenyl)-2-yl]acetate

[4'-Fluoro-3,3',5-trimethyl-(1,1'-biphenyl)-2-yl] carboxaldehyde (12.1 g, 50 mmoles) and methyl methylsulfinylmethyl sulfide (12.4 g, 100 mmoles) were dissolved in dry THF (100 mL). To this solution was added a 40% solution of Triton B in methanol (12 mL). The reaction solution was heated at a bath temperature of 60°C for four days under nitrogen. The reaction was partitioned between ether and water and the ether was extracted 3X with water, with 1N HCl, with water, then 2X with a saturated solution of sodium bicarbonate. The ether was dried ($MgSO_4$), filtered and the solvent was evaporated *in vacuo* to give 18.3 g of crude condensation product.

The condensation product was dissolved in ethanol (200 mL). Saturated ethanolic HCl (10 mL) was added and the mixture was refluxed for four hours. The reaction mixture was cooled and the solvent was evaporated *in vacuo*. The residue was dissolved in ether and washed with a saturated solution of sodium bicarbonate, dried ($MgSO_4$), filtered and the solvent evaporated *in vacuo* to leave

14.5 g of crude product. This material was purified by chromatography on a 100 mm diameter Still Column containing 1.1 kg of silica gel (230-400 mesh) eluting with 40% (v/v) methylene chloride in hexane to give 8.6 g of ethyl [4'-fluoro-3,3',5-trimethyl-(1,1'-biphenyl)-2-yl] acetate as an oil.
pmr ($DCCl_3$)δ 1.20 (3H, t); 2.3 (9H, s); 3.5 (2H, s); 4.1 (2H, q); 6.7-7.25 (5H, m).

Step B: Preparation of 2-[4'-fluoro-3,3',5-trimethyl-(1,1'-biphenyl)-2-yl]ethanol

Lithium aluminum hydride (6.49 g, 171.1 mmoles) was suspended in dry ether (900 mL). To this suspension was added dropwise a solution of ethyl [4'-fluoro-3,3',5-trimethyl-(1,1'-biphenyl)-2-yl]-acetate (25.7 g, 85.6 mmoles) from Step A dissolved in dry ether (250 mL). After the addition was complete, stirring was continued for one hour. In succession was added dropwise with vigorous stirring, 7 mL of water; 21 mL of 20% NaOH in water, then 7 mL of water. The ether containing the product was decanted from the solid; the solid was washed with ether; the combined ether solutions were dried ($MgSO_4$); filtered; and the solvent was evaporated *in vacuo* to give 21.5 g of 2-[4'-fluoro-3,3',5-trimethyl-(1,1'-biphenyl)-2-yl]ethanol.
pmr ($DCCl_3$)δ 1.2 (1H, t); 2.3 (6H, s); 2.4 (3H, s); 2.83 (2H, t); 3.45 (3H, t); 6.65-7.3 (5H, m).

Step C: Preparation of 2-[4'-fluoro-3,3',5-trimethyl-(1,1'-biphenyl)-2-yl]ethyl tosylate

p-Toluenesulfonyl chloride (23.8 g, 124.8 mmoles) was added to a stirred solution of 2-[4'-fluoro-3,3',5-trimethyl-(1,1'-biphenyl)-2-yl]ethanol (21.5 g, 83.2 mmoles) in dry pyridine (165 mL). The mixture was stirred at room temperature for two hours, cooled in an ice-water bath, and water (15 mL) was added dropwise and stirring was continued at room temperature for 2 hours. THe reaction mixture was dissolved in ether and successively extracted with water (3X); dilute ice cold HCl until washings were acidic; water; then saturated $NaHCO_3$ solution; dried ($MgSO_4$); filtered; and the solvent was evaporated to leave 30.92 g of solid 2-[4'-fluoro-3,3',5-trimethyl-(1,1'-biphenyl)-2-yl]ethyl tosylate, m.p. 77-81°C.

pmr ($DCCl_3$)δ 2.27 (9H, s); 2.4 (3H, s); 2.85 (2H, t); 3.82 (2H, t); 6.68-7.68 (9H, m).

<u>Step D</u>: Preparation of <u>2-[4'-fluoro-3,3',5-trimethyl-(1,1'-biphenyl)-2-yl]ethyl iodide</u>

A solution of 2-[4'-fluoro-3,3',5-trimethyl-(1,1'-biphenyl)-2-yl]ethyl tosylate (30.4 g, 73.69 mmoles) and sodium iodide (90 g, 0.6 mole) in acetone (900 mL) was heated under nitrogen with stirring in the dark with bath temperature of 50° for 48 hours. The solvent was evaporated *in vacuo* and the residue was partitioned between ether and water, washed successively with dilute aqueous sodium thiosulfate and water, dried ($MgSO_4$), filtered and the solvent was evaporated *in vacuo* to leave a tacky solid which was triturated with a small amount of hexane to give 20.29 g of 2-[4'-fluoro-3,3',5-trimethyl-(1,1'-biphenyl)-2-yl]ethyl iodide, m.p. 97-99°C.

pmr ($DCCl_3$)δ 2.22 (3H, s); 2.3 (6H, s); 3.04 (4H, s); 6.7-7.2 (5H, m).

Step E: Preparation of S-2-[4'-fluoro-3,3',5-trimethyl-(1,1'-biphenyl)-2-yl]ethyl isothiuronium iodide

A mixture of 2-[4'-fluoro-3,3',5-trimethyl-(1,1'-biphenyl)-2-yl]ethyl iodide (20.0 g, 54.3 mmoles) and thiourea (4.57 g, 60 mmoles) in ethanol was heated at 80° bath temperature with stirring under nitrogen. After 24 hours about one-half of the ethanol was evaporated in a stream of nitrogen. Heating was continued at 80° under $N_2$ with stirring for an additional 72 hours. Most of the solvent was evaporated in vacuo to leave a gum which was dissolved in ether (250 mL) and allowed to crystallize. The crystals were collected and dried in a vacuum oven to give 21.6 g of S-2-[4'-fluoro-3,3',5-trimethyl-(1,1'-biphenyl)-2-yl]ethyl isothiouronium iodide, m.p. 138-140°C.

Step F: Preparation of 2-[4'-fluoro-3,3',5-trimethyl-(1,1'-biphenyl)-2-yl]ethanethiol

S-2-[4'-Fluoro-3,3',5-trimethyl-(1,1'-biphenyl)-2-yl]ethyl isothiouronium iodide (0.44 g, 1 mmole) was suspended in ethanol (0.5 mL) and sodium hydroxide (0.6 mL of a 10% (w/v) solution of NaOH in water; 60 mg; 1.5 mmoles) was added. The mixture was refluxed for two hours (reactants went into solution then came out again as the sodium salt). Ethanol (0.5 mL) was added and the mixture was heated at 100° bath temperature for two hours more. The reaction mixture was cooled to room temperature and 2 mL of 1N HCl was added with stirring followed in 15 minutes by ether. The ether solution of the product was washed with water four times, dried ($MgSO_4$), filtered, and

the solvent was evaporated *in vacuo* to leave 0.24 g of 2-[4'-fluoro-3,3',5-trimethyl-(1,1'-biphenyl)-2-yl]ethanethiol, m.p. 96-97°C.
pmr ($DCCl_3$)δ 1.24 (1H, t); 2.34 (6H, s); 2.4 (3H, s); 2.3-3.04 (4H, m); 6.8-7.25 (5H, m).

Step G: Preparation of Methyl 7-[4'-fluoro-3,3',5-trimethyl-(1,1'-biphenyl)-2-yl]-3-oxo-5-thia-heptanoate

Sodium hydride (0.46 g, 9.62 mmoles of 50% in mineral oil) was suspended in dry DMF (15 mL) and 2-[4'-fluoro-3,3',5-trimethyl-(1,1'-biphenyl)-2-yl]-ethanethiol (2.4 g, 8.75 mmoles) was added in portions under nitrogen with stirring. The stirring was continued 15 minutes after the evolution of $H_2$ stopped. The reaction was cooled in an ice-water bath and methyl 4-chloroacetoacetate was added dropwise with a syringe through a rubber septum. The ice-water bath was removed and stirring was continued at ambient temperature for 2 hours. The reaction mixture was partitioned between ether and water. The ether layer was washed with water 4 times, dried ($MgSO_4$), filtered, and the solvent evaporated *in vacuo* to leave 3.6 g of crude product. This product was purified by flash chromatography on 80 X 260 mm silica gel Still column, eluting with 15% acetone in hexane to give 2.6 g of oily methyl 7-[4'-fluoro-3,3',5-trimethyl-(1,1'-biphenyl)-2-yl]-3-oxo-5-thia-heptanoate.
pmr ($DCCl_3$)δ 2.30 (6H, s); 2.35 (3H, s); 2.3-3.0 (4H, m); 3.1 (2H, s); 3.55 (2H, s); 3.7 (3H, s); 6.7-7.2 (5H, m).

Step H: Preparation of Methyl 7-[4'-fluoro-3,3',5-trimethyl-(1,1'-biphenyl)-2-yl]-3-hydroxy-5-thiaheptanoate

Sodium borohydride (0.12 g, 3.13 mmole) was added to a solution of methyl 7-[4'-fluoro-3,3',5-trimethyl-(1,1'-biphenyl)-2-yl]-3-oxo-5-thiaheptanoate (2.43 g, 6.25 mmole) in methanol (30 mL) with stirring and cooling in an ice-water bath. After 15 minutes the reaction was dissolved in ether (200 mL) and extracted 4 times with water, dried ($MgSO_4$), filtered and the solvent was evaporated to give the crude product. The crude product was purified by flash chromatography on a Still silica gel column (80 x 200 mm) eluting with 23% acetone in hexane to give 2.08 g of oily methyl 7-[4'-fluoro-3,3',5-trimethyl-(1,1'-biphenyl)-2-yl]-3-hydroxy-5-thiaheptanoate; high resolution mass spectrum calculated and found = 390.1658; elemental composition = $C_{22}H_{27}FO_3S$. pmr ($DCCl_3$)$\delta$ 2.22 (6H, s); 2.3 (3H, s); 2.35-3.1 (8H, m); 3.7 (3H, s); 3.6-4.0 (1H, m); 6.65-7.2 (5H, m).

Step I: Preparation of Sodium 7-[4'-fluoro-3,3',5-trimethyl-(1,1'-biphenyl)-2-yl]-3-hydroxy-5-thiaheptanoate

Methyl 7-[4'-fluoro-3,3',5-trimethyl(1,1'-biphenyl)-2-yl]-3-hydroxy-5-thiaheptanoate (4 mg, .01024 mmole) is dissolved in 0.1 mL of ethanol. To this solution is added 102.4 microliters of 0.1N NaOH (0.01024 mmoles) with gentle shaking and then let stand for 2 hours at room temperature with occasional gentle shaking. The solution of the sodium salt of the hydrolyzed ester is diluted with water to 1 mL.

This solution is lyophilized to give sodium 7-[4'-fluoro-3,3',5-trimethyl-(1,1'-biphenyl)-2-yl]-3-hydroxy-5-thiaheptanoate.

Step J: Preparation of 7-[4'-fluoro-3,3',5-trimethyl-(1,1'-biphenyl)-2-yl]-3-hydroxy-5-thiaheptanoic acid

The aqueous solution of the sodium salt from Step I is treated with 102.4 microliters of 0.1N HCl and extracted with ether 3 times. The combined ether extracts are dried ($MgSO_4$), and filtered to give an ether solution of 7-[4'-fluoro-3,3',5-trimethyl-(1,1'-biphenyl)-2-yl]-3-hydroxy-5-thiaheptanoic acid. The solvent is evaporated *in vacuo* to give title.

EXAMPLE 2

7-[2,4-dichloro-6-(4-fluorobenzyloxy)phenyl]-3-hydroxy-5-thiaheptanoic acid

Following the procedure substantially as described in Example 1, Steps A through J, but substituting for the [4'-fluoro-3,3'5-trimethyl-(1,1'-biphenyl)-2-yl] carboxaldehyde used in Step A thereof, a similar amount of 2,4-dichloro-6-(4-fluorobenzyloxy)benzaldehyde there are produced in sequence:

Step A: Ethyl 2,4-dichloro-6-(4-fluorobenzyloxy)-phenyl acetate; m.p. 67-68°C.

Calc. for $C_{17}H_{15}Cl_2FO_3$: C, 57.16; H, 4.23.
Found: C, 57.12; H, 4.33.

Step B: 2-[2,4-Dichloro-6-(4-fluorobenzyloxy)phenyl]-ethanol;
pmr ($DCCl_3$): δ 1.75 (1H, m); 3.12 (2H, t); 3.75 (2H, m); 5.00 (2H, s); 6.7-7.6 (6H, m).

Step C: 2-[2,4-dichloro-6-(4-fluorobenzyloxy)phenyl]-ethyl tosylate; m.p. 100-101.5°C.
Calc. for $C_{22}H_{19}Cl_2FO_4S$: C, 56.29; H, 4.08.
Found: C, 56.31; H, 4.06.

Step D: 2-[2,4-Dichloro-6-(4-fluorobenzyloxy)phenyl]-ethyl iodide; m.p. 60-62°C.
pmr ($DCCl_3$): δ 3.25 (4H, s); 5.02 (2H, s); 6.8-7.6 (6H, m).

Step E: S-2-[2,4-dichloro-6-(4-fluorobenzyloxy)-phenyl]ethyl isothiouronium iodide; m.p. 175-177°C.
Calc. for $C_{16}H_{16}Cl_2FIN_2OS$: C, 38.42; H, 3.02; N, 5.60
Found: C, 38.11; H, 3.21; N, 5.61.

Step F: 2-[2,4-Dichloro-6-(4-fluorobenzyloxy)phenyl]-ethane thiol; m.p. 56-58°C.
pmr ($DCCl_3$): δ 1.35 (1H, t); 2.3-3.2 (4H, m); 5.0 (2H, s); 6.6-7.5 (6H, m).

Step G: Methyl 7-[2,4-dichloro-6-(4-fluorobenzyl-oxy)phenyl]-3-oxo-5-thiaheptanoate;
pmr ($DCCl_3$): δ 2.4-3.2 (4H, m); 3.3 (2H, s); 3.54 (2H, s); 3.7 (3H, s); 5.0 (2H, s); 6.7-7.5 (6H, m).

Step H: Methyl 7-[2,4-dichloro-6-(4-fluorobenzyloxy)-phenyl]-3-hydroxy-5-thiaheptanoate;

Calc. for $C_{30}H_{21}Cl_2FO_4S$: C, 53.70; H, 4.73;
Found: C, 53.77; H, 4.84.

pmr ($DCCl_3$): $\delta$ 2.45-2.73 (6H, m); 3.06 (2H, q); 3.72 (3H, s); 4.08 (1H, m); 6.83 (1H, d); 7.03 (1H, d); 7.11 (2H, t); 7.38 (2H, q).

Step I: Sodium 7-[2,4-dichloro-6-(4-fluorobenzyloxy)phenyl]-3-hydroxy-5-thiaheptanoate acid; and

Step J: 7-[2,4-Dichloro-6-(4-fluorobenzyloxy)phenyl]-3-hydroxy-5-thiaheptanoic acid.

## EXAMPLE 3

6-[4'-fluoro-3,3',5-trimethyl-(1,1'-biphenyl)-2-yl]-3-hydroxy-5-thiahexanoic Acid

Step A: Preparation of 4'-Fluoro-3,3',5-trimethyl-(1,1'-biphenyl)methanol

4'Fluoro-3,3',5-trimethyl-(1,1'-biphenyl)-2-yl carboxaldehyde (2.90 g, 12 mmoles) is suspended in ethanol (20 mL) and cooled with stirring in an ice-water bath. Sodium borohydride (0.45 g, 12 mmoles) is added and the cooling bath removed.

Stirring is continued at ambient temperature for 1 hour. The mixture is cooled to 0°, treated with excess ammonium chloride, partioned between ether and water. The water was extracted with ether twice and the combined ether extracts were washed with water, dried ($MgSO_4$), filtered and evaporated to dryness to leave 2.77 g of 4'-fluoro-3,3',5-trimethyl-(1,1'-biphenyl)methanol, m.p. 74-75°C. A sublimed sample had m.p. of 102-103°C.

Following the procedures substantially as described in Example 1, Steps C through J, but substituting for the 2-[4'-fluoro-3,3',5-trimethyl-(1,1'-biphenyl)-2-yl]ethanol used as a starting material in Step C, an equivalent amount of the corresponding substituted methanol described in Step A of this Example 3, there are produced in sequence the intermediates and products of Steps B through I as follows:

<u>Step B</u>: [4'-fluoro-3,3',5-trimethyl-(1,1'-biphenyl)-2-yl)]methyl tosylate;

<u>Step C</u>: 2-[4'-fluoro-3,3',5-trimethyl-(1,1'-biphenyl-2-yl)]methyl iodide;

<u>Step D</u>: S-[4'-fluoro-3,3',5-trimethyl-(1,1'-biphenyl)-2-yl]methyl isothiuronium iodide;

<u>Step E</u>: [4'-fluoro-3,3',5-trimethyl-(1,1'-biphenyl)-2-yl]methanethiol;

<u>Step F</u>: Methyl 6-[4'-fluoro-3,3',5-trimethyl-(1,1'-biphenyl)-2-yl]-3-oxo-5-thiahexanoate;

<u>Step G</u>: Methyl 6-[4'-fluoro-3,3',5-trimethyl-(1,1'-biphenyl)-2-yl]-3-hydroxy-5-thiahexanoate;

Step H: Sodium 6-[4'-fluoro-3,3',5-trimethyl-(1,1'-biphenyl)-2-yl]-3-hydroxy-5-thiahexanoate; and

Step I: 6-[4'-fluoro-3,3',5-trimethyl-(1,1'-biphenyl)-2-yl]-3-hydroxy-5-thiahexanoic acid.

EXAMPLE 4

8-[4'-Fluoro-3,3',5-trimethyl-(1,1'-biphenyl)-2-yl]-3-hydroxy-5-thiaoctanoic Acid

Step A: Preparation of Ethyl E-3-[4'-fluoro-3,3',5-trimethyl-(1,1'-biphenyl)-2-yl]-propenoate

Sodium hydride (50% in mineral oil; 12.0 g, 0.25 mole) is added in portions to ethyl acetate (200 mL) with stirring and cooling to -10°C. A solution of 4'-fluoro-3,3',5-trimethyl-(1,1-biphenyl)-2-yl-carboxaldehyde (60.57 g, 0.25 mole) in 50 ml of ethyl acetate is added dropwise over 30 minutes with cooling to -5°C. The bath is removed and the reaction mixture allowed to warm spontaneously and is stirred at room temperature overnight. The reaction mixture is treated with acetic acid (excess) and partitioned between ether and water. The ether layer is extracted with aqueous sodium bicarbonate, water, dried ($MgSO_4$), filtered and the solvent is evaporated *in vacuo* to give ethyl E-3-[4'-fluoro-3,3',5-trimethyl-(1,1'-biphenyl)-2-yl]-propenoate.

Step B: Preparation of 3-[4'-fluoro-3,3',5-trimethyl-(1,1'-biphenyl)-2-yl]-propan-1-ol

A solution of ethyl E-3-[4'-fluoro-3,3',5-trimethyl-(1,1'-biphenyl)-2-yl]-propenoate (55.61 g, 0.178 mole) in ether (500 mL) is added to a mechanically stirred suspension of lithium aluminum

hydride (9.6 g, 0.25 mole) in ether (500 mL) with cooling in an ice bath and stirred overnight at room temperature. The reaction mixture is cooled in an ice-water bath and 10 mL of water is added dropwise over 30 minutes with vigorous stirring. Aqueous sodium hydroxide (30 ml of 20% v/v) is added over 15 minutes with vigorous stirring. Water (10 ml) is added dropwise with vigorous stirring. The product, dissolved in ether, is decanted from the salts. The salts are washed with ether by decantation and the combined ether portions are dried ($MgSO_4$), filtered and evaporated _in vacuo_ to leave 3-[4'-fluoro-3,3',5-trimethyl-(1,1'-biphenyl)-2-yl]propan-1-ol.

Following the procedures substantially as described in Example 1, Steps C through J, but substituting for the 2-[4'-fluoro-3,3',5-trimethyl-(1,1'-biphenyl)-2-yl] ethanol used as starting material in Step C, an equivalent amount of the corresponding substituted propan-1-ol described in Step B of this Example 4, there are produced in sequence, the intermediates and products of Steps C through J as follows:

_Step C_: 3-[4'-fluoro-3,3',5-trimethyl-(1,1'-biphenyl)-2-yl]propan-1-yl-tosylate;

_Step D_: 3-[4'-fluoro-3,3',5-trimethyl-(1,1'-biphenyl)-2-yl)]propan-1-yl-iodide;

_Step E_: S-3-[4'-fluoro-3,3',5-trimethyl-(1,1'-biphenyl)-2-yl]propan-1-yl-isothiuronium iodide;

_Step F_: 3-[4'-fluoro-3,3',5-trimethyl-(1,1'-biphenyl)-2-yl]propan-1-thiol;

_Step G_: Methyl 8-[4'-fluoro-3,3',5-trimethyl-(1,1'-biphenyl)-2-yl]-3-oxo-5-thiaoctanoate;

Step H: Methyl 8-[4'-fluoro-3,3',5-trimethyl-(1,1'-biphenyl)-2-yl]-3-hydroxy-5-thiaoctanoate;

Step I: Sodium 8-[4'-fluoro-3,3',5-trimethyl-(1,1'-biphenyl)-2-yl]-3-hydroxy-5-thiaoctanoate; and

Step J: 8-[4'-fluoro-3,3',5-trimethyl-(1,1'-biphenyl)-2-yl]-3-hydroxy-5-thiaoctanoic acid.

EXAMPLE 5

E-8-[4'-Fluoro-3,3',5-trimethyl-(1,1'-biphenyl)-2-yl]-3-hydroxy-5-thiaocta-7-enoic Acid

Step A: Preparation of E-3-[4'-fluoro-3,3',5-trimethyl-(1,1'-biphenyl)-2-yl]-propene-1-ol

Ethanol (0.266 g) is added to a solution of lithium aluminum hydride (2.2 g, 57.97 mmoles) in dry ether 100 mL. This solution is added dropwise to a solution of ethyl E-3-[4'-fluoro-3,3',5-trimethyl-(1,1'-biphenyl)-2-yl]-propenoate (36.21 g, 115.94 mmoles) dissolved in 100 mL of dry ether slowly over 30 minutes while cooling in an ice-water bath. The reaction is stirred 1 hour then treated with the cautious dropwise addition of water (3 mL) with vigorous stirring, 20% aqueous sodium hydroxide (6 ml) dropwise with vigorous stirring, and water (3 mL) dropwise with vigorous stirring. The ether is decanted from the salts, the salts are washed with ether and the combined ether extracts are dried ($MgSO_4$), filtered and the solvent evaporated in vacuo to give E-3-[4'-fluoro-3,3',5-trimethyl-(1,1'-biphenyl)-2-yl]-propen-1-ol.

Following the procedures substantially as described in Example 1, Steps C through J, but substituting for the 2-[4'-fluoro-3,3',5-trimethyl-

(1,1'-biphenyl)-2-yl]-ethanol used as starting material in Step C, an equivalent amount of the corresponding substituted 2-propen-1-ol described in Step A of this Example 5, there are produced in sequence, the intermediates and products of Steps B through I as follows:

<u>Step B</u>: 3-[4'-fluoro-3,3',5-trimethyl-(1,1'-biphenyl)-2-yl]-2-propen-1-yl-tosylate;

<u>Step C</u>: 3-[4'-fluoro-3,3',5-trimethyl-(1,1'-biphenyl)-2-yl]-2-propen-1-yl-iodide;

<u>Step D</u>: S-3-[4'-fluoro-3,3',5-trimethyl-(1,1'-biphenyl)-2-yl]-2-propen-1-yl-isothiouronium iodide;

<u>Step E</u>: 3-[4'-fluoro-3,3',5-trimethyl-(1,1'-biphenyl)-2-yl]-2-propen-1-thiol;

<u>Step F</u>: Methyl 8-[4'-fluoro-3,3',5-trimethyl-(1,1'-biphenyl)-2-yl]-3-oxo-5-thiaoct-7-enoate;

<u>Step G</u>: Methyl 8-[4'-fluoro-3,3',5-trimethyl-(1,1'-biphenyl)-2-yl]-3-hydroxy-5-thiaoct-7-enoate;

<u>Step H</u>: Sodium 8-[4'-fluoro-3,3',5-trimethyl-(1,1'-biphenyl)-2-yl]-3-hydroxy-5-thiaoct-7-enoate; and

<u>Step I</u>: 8-[4'-fluoro-3,3',5-trimethyl-(1,1'-biphenyl)-2-yl]-3-hydroxy-5-thiaoct-7-enoic acid.

Employing the procedures substantially as described in Examples 1 and 2 (E = $-CH_2CH_2-$), 3 (E = $-CH_2-$), 4 (E = $-(CH_2)_3-$) and 5 (E = $-CH{=}CH_2CH_2-$) but substituting, for the particular benzaldehyde derivative used as starting material in each case, the benzaldehyde derivatives described in Table I, there are produced the 3-hydroxy-5-thia- -Z-$C_{6-8}$ acid, salts and esters also described in Table I:

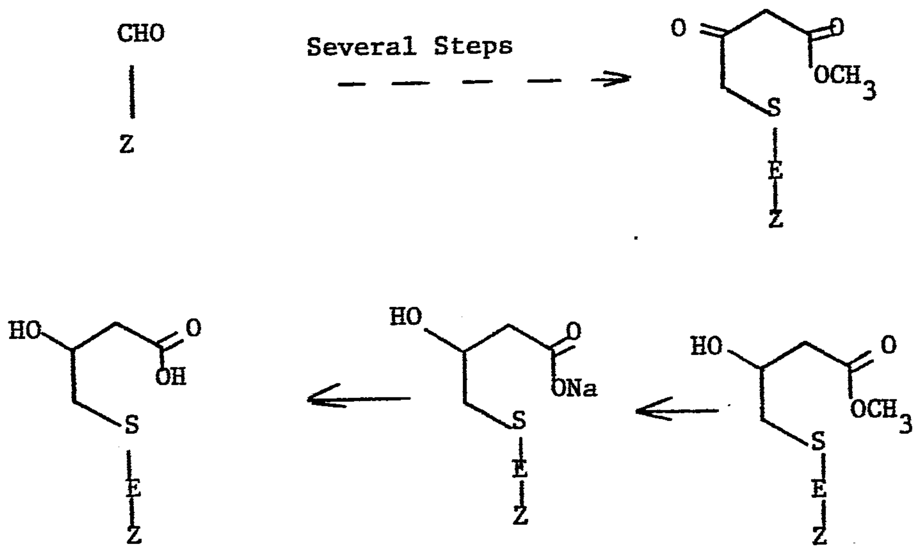


TABLE I

E=$-(CH_2)_2-$; $-CH_2-$; $-(CH_2)_3$; $-CH=CH-CH_2-$

Z =

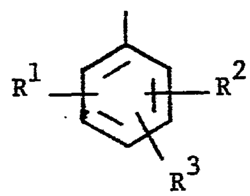

| $R^1$ | $R^2$ | $R^3$ |
|---|---|---|
| 6-(3',5'-dimethylphenyl) | 2-$CH_3$ | 4-$CH_3$ |
| 6-(4'-fluorophenyl) | 2-Cl | 4-Cl |
| 6-(3',4'-dichlorophenyl) | 2-Cl | 4-Cl |
| 6-(3'-methyl-4'-fluorophenyl) | 2-Cl | 4-Cl |
| 6-(3',4'-dichlorophenyl) | 2-$CH_3$ | 4-$CH_3$ |
| 6-(3',4'-dichlorophenyl) | 2-$CH_3$ | 5-$CH_3$ |
| 6-(4'-fluorophenyl) | 2-$CH_3$ | 4-$CH_3$ |
| 6-(3'-methyl-4'-fluorophenyl) | 2-$CH_3$ | 4-Cl |
| 6-benzyloxy | 2-Cl | 4-Cl |
| 6-benzyloxy | 2-$CH_3$ | 4-$CH_3$ |
| 6-(4-fluorobenzyloxy | 2-$CH_3$ | 4-Cl |

TABLE I (Cont'd.)

| | | |
|---|---|---|
| 6-n-pentyloxy- | 2-Cl | 4-Cl |
| 6-(2,4-dichlorobenzyloxy)- | 2-Cl | 4-Cl |
| 6-allyloxy- | 2-Cl | 4-Cl |
| 6-(3-phenyl-l-propoxy)- | 2-Cl | 4-Cl |
| 6-cinnamyloxy- | 2-Cl | 4-Cl |
| 6-(4-methylbenzyloxy)- | 2-Cl | 4-Cl |
| 6-(4-methoxybenzyloxy)- | 2-Cl | 4-Cl |
| 6-hexafluorobenzyloxy- | 2-Cl | 4-Cl |
| H | 2-Cl | 4-Cl |
| H | 2-$CH_3$ | 4-$CH_3$. |
| 6-phenyl- | 2-Cl | 4-Cl |
| 6-phenoxy- | 2-Cl | 4-Cl |
| 6-(4-trifluoromethyl-benzyloxy)- | 2-Cl | 4-Cl |
| 6-(4-methoxyphenyl)- | 2-Cl | 4-Cl |
| 6-(4-methylphenyl)- | 2-Cl | 4-Cl |
| 6-hydroxy | 2-Cl | 4-Cl |
| 6-(4-fluorophenoxy)- | 2-Cl | 4-Cl |
| 6-(4-methylphenoxy)- | 2-Cl | 4-Cl |
| 6-(4-methoxyphenoxy)- | 2-Cl | 4-Cl |
| 6-cyclohexylmethoxy- | 2-Cl | 4-Cl |
| 6-benzyloxy- | 2-Cl | 4-Cl |
| 6-acetoxy- | 2-Cl | 4-Cl |
| 6-benzoyloxy- | 2-Cl | 4-Cl |
| 6-(4-trifluoromethylphenyl)- | 2-Cl | 4-Cl |
| 6-adanantylmethoxy- | 2-Cl | 4-Cl |
| 6-(2-chloro-l-ethoxy)- | 2-Cl | 4-Cl |
| 6-(4-acetylphenoxy)- | 2-Cl | 4-Cl |
| 6-(4-trifluoromethylphenoxy)- | 2-Cl | 4-Cl |
| 6-(4-acetylbenzyloxy)- | 2-Cl | 4-Cl |
| 6-(2-chloro-l-ethyl)- | 2-Cl | 4-Cl |
| 6-(4-acetylphenyl)- | 2-Cl | 4-Cl |

Z = 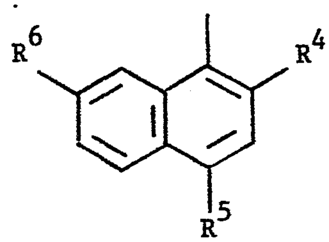

| $R^4$ | $R^5$ | $R^6$ |
|---|---|---|
| -Cl | -Cl | -Cl |
| $-CH_3$ | $-CH_3$ | $-CH_3$ |
| $-CH_3$ | $-CH_3$ | -F |
| $-CH_3$ | $-CH_3$ | -Cl |

EXAMPLE 6

7-[4'-fluoro-3,3',5-trimethyl-(1,1'-biphenyl)-2-yl]-3-hydroxy-5-oxo-5-thiaheptanoic acid

Step A: Preparation of Methyl 7-[4'-fluoro-3,3',5-trimethyl-(1,1'-biphenyl)-2-yl]-3,5-dioxo-5-thiaheptanoate

m-Chloroperbenzoic acid (1.39 g, 8.03 mmoles of 80-85% reagent) was added in small portions over 15 minutes to a stirred solution of methyl 7-[4'-fluoro-3,3',5-trimethyl-(1,1'-biphenyl)-3-oxo-5-thiaheptanoate (2.6 g, 6.69 mmoles) in 40 mL of $CH_2Cl_2$ which had been cooled to -78°C in a dry ice-acetone bath then at -78°C allowed to warm to room temperature after 30 minutes. The reaction was dissolved in ether and extracted with a saturated solution of sodium bicarbonate, dried ($MgSO_4$), filtered, and the solvent was evaporated in vacuo to give 2.7 g of methyl 7-[4'-fluoro-3,3',5-trimethyl-(1,1'-biphenyl)-2-yl]-3,5-dioxo-5-thiaheptanoate

which contains a trace of sulfone. This was used in the next step without further purification.
pmr ($DCCl_3$)δ 2.3 (6H, s); 2.4 (3H, s); 2.6-3.2 (4H, m); 3.2-3.5 (2H, m); 3.57 (2H, s); 3.72 (3H, s); 6.73-7.25 (5H, m).

Step B: Preparation of Methyl 7-[4'-fluoro-3,3',5-trimethyl-(1,1'-biphenyl)-2-yl]-3-hydroxy-5-oxo-5-thiaheptanoate isomer A and isomer B

Sodium borohydride (0.51 g, 13.35 mmoles) was added in divided portions to a stirred suspension of methyl 7-[4'-fluoro-3,3',5-trimethyl-(1,1'-biphenyl)-2-yl]-3,5-dioxo-5-thiaheptanoate (2.7 g, 6.68 mmoles) in 80 mL of methanol cooled in an ice-water bath. When the addition was complete, the cooling bath was removed and stirring was continued for 1 hour at ambient temperature at which time solution was complete. The reaction mixture was partitioned between ether and water. The water was extracted with ether and the combined ether extracts were washed 2X with water containing a little NaCl, dried ($MgSO_4$), filtered and evaporated *in vacuo* to give 2.6 g of a mixture of sulfoxide, alcohols and impurities. The mixture was flash chromatographed on a Still silica column (60 x 180 mm) eluting with 25% (v/v) acetone in methylene chloride. The first compound to emerge from the column was isomer A which was 96% isomerically pure, but contained another impurity which was removed by HPLC on a silica column eluting with 97% hexane and 3% 2-propanol (v/v) to give 120 mg of methyl 7-[4'-fluoro-3,3',5-trimethyl-(1,1'-biphenyl)-2-yl]-3-hydroxy-5-oxo-5-thiaheptanoate, isomer A, m.p. 98-100°C.

Calc. for $C_{22}H_{27}FO_4S$: C, 65.00; H, 6.69
Found: C, 65.20; H, 6.93.

Isomer A pmr ($DCCl_3$) $\delta$ 2.31 (6H, s); 2.38 (3H, s); 2.5 (1H, dd); 2.55 (2H, m); 2.64 (1H, dd); 2.65-2.8 (2H, m); 2.88-2.96 (2H, m); 3.73 (3H, s); 3.98 (1H, d); 4.48-4.58 (1H, m); 6.86 (1H, s); 7.0-7.1 (4H, m).

The second compound to emerge from the column was methyl 7-[4'-fluoro-3,3',5-trimethyl-(1,1'-biphenyl)-2-yl]-3-hydroxy-5-oxo-5-thiaheptanoate, isomer B, 96% isomer purity, m.p. 114-116°C; high resolution mass spectrum, parent(-OH) = 389.1569; ($C_{22}H_{26}O_3SF$)

Isomer B pmr ($DCCl_3$) $\delta$ 2.31 (3H, s); 2.32 (3H, s); 2.41 (3H, s); 2.43 (1H, dd); 2.54 (2H, m); 2.57 (1H, dd); 2.70 (2H, t); 2.87-3.06 (2H, m); 3.73 (3H, s); 3.75 (1H, d); 4.54 (1H, m); 6.85 (1H, s); 7.0-7.1 (4H, m).

<u>Step C</u>: <u>Preparation of Sodium 7-[4'-fluoro-3,3',5-trimethyl-(1,1'-biphenyl)-2-yl]-3-hydroxy-5-oxo-5-thiaheptanoate (Isomers A and B)</u>

The ester, isomers A and B, from Step B are converted to their sodium salts by the procedure described in Example 1, Step I.

<u>Step D</u>: <u>Preparation of 7-[4'-fluoro-3,3',5-trimethyl-(1,1'-biphenyl)-2-yl]-3-hydroxy-5-oxo-5-thiaheptanoic acid (Isomers A and B)</u>

The title free acid, isomers A and B, are prepared by the process described in Example 1, Step J.

EXAMPLE 7

7-[2,4-Dichloro-6-(4-fluorobenzyloxy)phenyl]-3-hydroxy-5-oxo-5-thiaheptanoic acid

Following the procedure of Example 3, Steps A through D, but substituting for the methyl 7-[4'-fluoro-3,3',5-trimethyl-(1,1'-biphenyl)-2-yl]-3-oxo-5-thiaheptanoate used in Step A thereof, a similar amount of methyl 7-[2,4-dichloro-6-(4-fluorobenzyloxy)phenyl]-3-oxo-5-thiaheptanoate, there were produced in sequence:

Step A: Methyl 7-[2,4-dichloro-6-(4-fluorobenzyloxy)-phenyl]-3,5-dioxo-5-thiaheptanoate

Step B: Methyl 7-[2,4-dichloro-6-(4-fluorobenzyloxy)-phenyl]-3-hydroxy-5-oxo-5-thiaheptanoate

Isomer Mixture:

Calc. for $C_{20}H_{21}Cl_2FO_5S$: C, 51.84; H, 4.57

Found: C, 51.76; H, 4.65

Isomer A: m.p. 119-120°C
molecular weight by high resolution mass spec. 462.0470.

Isomer B: a gum
molecular weight by high resolution mass spec. 462.0470.

Step C: Sodium 7-[2,4-dichloro-6-(4-fluorobenzyloxy)-phenyl]-3-hydroxy-5-oxo-5-thiaheptanoate, (Isomers A and B)

Step D: 7-[2,4-Dichloro-6-(4-fluorobenzyloxy)phenyl]-3-hydroxy-5-oxo-5-thiaheptanoic acid (Isomers A and B)

Employing the procedure substantially as described in Example 6, Steps A through D but substituting for the methyl 7-[4'-fluoro-3,3',5-trimethyl-(1,1'-biphenyl)-2-yl]-3-oxo-5-thiaheptanoate used in Step A thereof, the 3-oxo-5-thia-$C_{6-8}$-acid esters described in Table II, there are produced the 3-hydroxy-5-oxo-5-thia-$C_{6-8}$ acids also described in Table II:

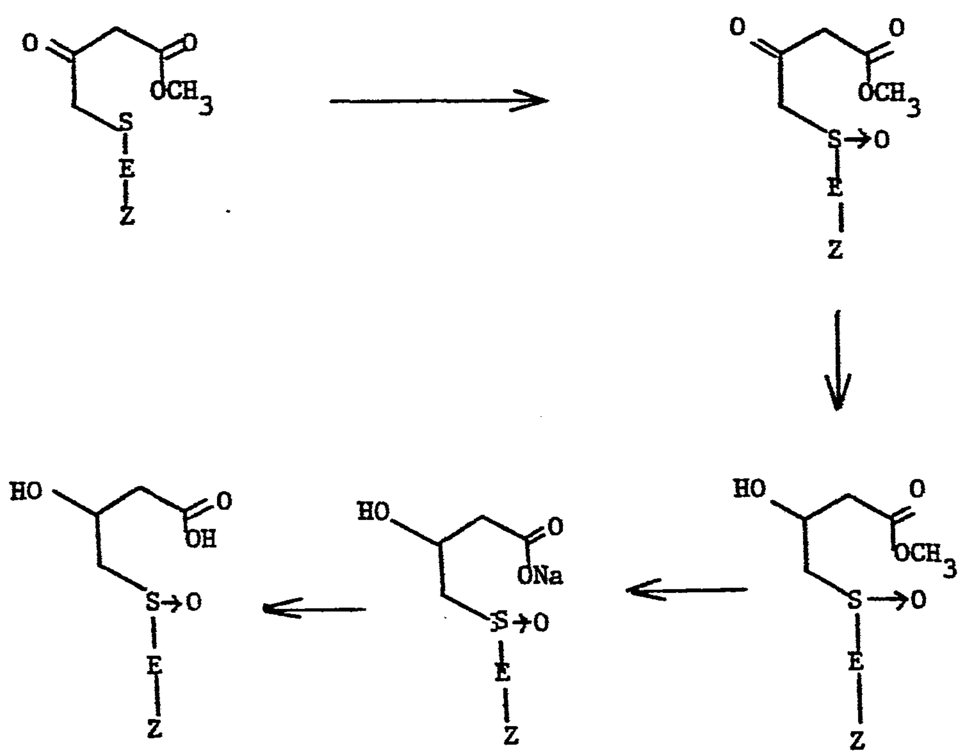

TABLE II

E=$-(CH_2)_2-$; $-CH_2$; $-(CH_2)_3-$; $-CH{=}CH_2CH_2-$

Z =

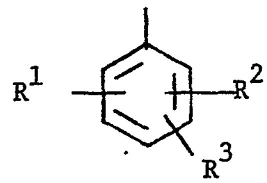

| $R^1$ | $R^2$ | $R^3$ |
|---|---|---|
| 6-(3',5'-dimethylphenyl) | 2-$CH_3$ | 4-$CH_3$ |
| 6-(4'-fluorophenyl) | 2-Cl | 4-Cl |
| 6-(3',4'-dichlorophenyl) | 2-Cl | 4-Cl |
| 6-(3'-methyl-4'-fluorophenyl) | 2-Cl | 4-Cl |
| 6-(3',4'-dichlorophenyl) | 2-$CH_3$ | 4-$CH_3$ |
| 6-(3',4'-dichlorophenyl) | 2-$CH_3$ | 5-$CH_3$ |
| 6-(4'-fluorophenyl) | 2-$CH_3$ | 4-$CH_3$ |
| 6-(3'-methyl-4'-fluorophenyl) | 2-$CH_3$ | 4-Cl |
| 6-benzyloxy | 2-Cl | 4-Cl |
| 6-benzyloxy | 2-$CH_3$ | 4-$CH_3$ |
| 6-(4-fluorobenzyloxy | 2-$CH_3$ | 4-Cl |
| 6-n-pentyloxy- | 2-Cl | 4-Cl |
| 6-(2,4-dichlorobenzyloxy)- | 2-Cl | 4-Cl |
| 6-allyloxy- | 2-Cl | 4-Cl |
| 6-(3-phenyl-1-propoxy)- | 2-Cl | 4-Cl |
| 6-cinnamyloxy- | 2-Cl | 4-Cl |
| 6-(4-methylbenzyloxy)- | 2-Cl | 4-Cl |
| 6-(4-methoxybenzyloxy)- | 2-Cl | 4-Cl |
| 6-hexafluorobenzyloxy- | 2-Cl | 4-Cl |
| H | 2-Cl | 4-Cl |
| H | 2-$CH_3$ | 4-$CH_3$ |
| 6-phenyl- | 2-Cl | 4-Cl |
| 6-phenoxy- | 2-Cl | 4-Cl |

TABLE II (Cont'd.)

| | | |
|---|---|---|
| 6-(4-trifluoromethyl-benzyloxy)- | 2-Cl | 4-Cl |
| 6-(4-methoxyphenyl)- | 2-Cl | 4-Cl |
| 6-(4-methylphenyl)- | 2-Cl | 4-Cl |
| 6-hydroxy | 2-Cl | 4-Cl |
| 6-(4-fluorophenoxy)- | 2-Cl | 4-Cl |
| 6-(4-methylphenoxy)- | 2-Cl | 4-Cl |
| 6-(4-methoxyphenoxy)- | 2-Cl | 4-Cl |
| 6-cyclohexylmethoxy- | 2-Cl | 4-Cl |
| 6-benzyloxy- | 2-Cl | 4-Cl |
| 6-acetoxy- | 2-Cl | 4-Cl |
| 6-benzoyloxy- | 2-Cl | 4-Cl |
| 6-(4-trifluoromethylphenyl)- | 2-Cl | 4-Cl |
| 6-adamantylmethoxy- | 2-Cl | 4-Cl |
| 6-(2-chloro-1-ethoxy)- | 2-Cl | 4-Cl |
| 6-(4-acetylphenoxy)- | 2-Cl | 4-Cl |
| 6-(4-trifluoromethylphenoxy)- | 2-Cl | 4-Cl |
| 6-(4-acetylbenzyloxy)- | 2-Cl | 4-Cl |
| 6-(2-chloro-1-ethyl)- | 2-Cl | 4-Cl |
| 6-(4-acetylphenyl)- | 2-Cl | 4-Cl |

R = 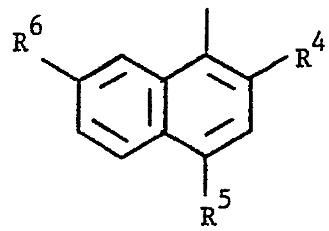

| $R^4$ | $R^5$ | $R^6$ |
|---|---|---|
| -Cl | -Cl | -Cl |
| $-CH_3$ | $-CH_3$ | $-CH_3$ |
| $-CH_3$ | $-CH_3$ | -F |
| $-CH_3$ | $-CH_3$ | -Cl |

EXAMPLE 8

7-[2,4-dichloro-6-(4-fluorobenzyloxy)phenyl]-3-hydroxy-5,5-dioxo-5-thiaheptanoic acid

Step A: Preparation of Methyl 7-[2,4-dichloro-6-(4-fluorobenzyloxy)phenyl]-3,5,5-trioxo-5-thiaheptanoate

Methyl 7-[2,4-dichloro-6-(4-fluorobenzyloxy)-phenyl]-3-oxo-5-thiaheptanoate (0.6 g, 1.4 mmole) was dissolved in methylene chloride (5 mL) and cooled in an ice bath. m-Chloroperbenzoic acid (0.67 g, 3.88 mmole of 85% tech. grade) was added all at once. After stirring for 15 minutes, the ice-bath was removed and stirring was continued at ambient temperature for three hours. The reaction was dissolved in ether (150 mL) and extracted two times

with dilute sodium bicarbonate solution, then shaken vigorously with excess aqueous citric acid solution. The ether was dried ($MgSO_4$) filtered, and the solvent was evaporated to leave 0.55 g of methyl 7-[2,4-dichloro-6-(4-fluorobenzyloxy)phenyl]-3,5,5-trioxy-5-thiaheptanoate.

pmr ($DCCl_3$) 3.10 (2H, s); 3.15 (2H, s); 3.80 (3H, s); 3.81 (2H, s); 4.24 (4H, s); 5.05 (2H, s); 3.35 (1/3 H, s); 6.8-7.6 (6H, m).

<u>Step B</u>: <u>Preparation of Methyl 7-[2,4-dichloro-6-(4-fluorobenzyloxy)phenyl]-3-hydroxy-5,5-dioxo-5-thia-heptanoate</u>

Sodium borohydride (0.040 g, 1.06 mmoles) was added to a solution of methyl 7-[2,4-dichloro-6-(4-fluorobenzyloxy)phenyl]-3,5,5-trioxo-5-thia-heptanoate (0.252 g, 0.528 mmoles) in 6 mL of methanol, stirred and cooled in an ice-water bath. The reaction was stirred for 15 minutes, then 1N HCl (4.24 mL, 4.24 mmoles) was added dropwise. The reaction mixture was partitioned between ether and water. The ether layer was washed with water twice, dried ($MgSO_4$), filtered, and the solvent was evaporated to leave crude product which after flash chromatography by eluting with 25% acetone in hexane gave 0.120 g of methyl 7-[2,4-dichloro-6-(4-fluorobenzyloxy)phenyl]-3-hydroxy-5,5-dioxo-5-thiaheptanoate, m.p. 97-98.5°C

Calc. for $C_{20}H_{21}Cl_2FO_6S$: C, 50.11; H, 4.42

Found: C, 49.81; H, 4.57.

pmr ($DCCl_3$)$\delta$ 2.56 (2H, d); 3.0-3.5 (6H, m); 3.73 (3H, s); 4.58 (1H, m); 5.05 (2H, s); 6.86 (1H, d); 7.06 (1H, d); 7.11 (2H, t); 7.42 (2H, q).

Step C: Preparation of Sodium 7-[2,4-dichloro-6-(4-fluorobenzyloxy)phenyl]-3-hydroxy-5,5-dioxo-5-thiaheptanoate

The ester from Step B is converted to the sodium salt by the procedure described in Example 1, Step I.

Step D: Preparation of 7-[2,4-dichloro-6-(4-fluorobenzyloxy)phenyl]-3-hydroxy-5,5-dioxo-5-thiaheptanoic acid

The title free acid is prepared by the process described in Example 1, Step J.

Employing the procedure substantially as described in Example 8, Steps A through D, but substituting for the methyl 7-[2,4-dichloro-6-(4-fluorobenzyloxy)phenyl]-3-oxo-5-thiaheptanoate used in Step A thereof, the 3-oxo-5-thia-$C_{6-8}$ acid esters described in Table III, there are produced the 3-hydroxy-5,5-dioxo-5-thia-$C_{6-8}$ acids also described in Table III.

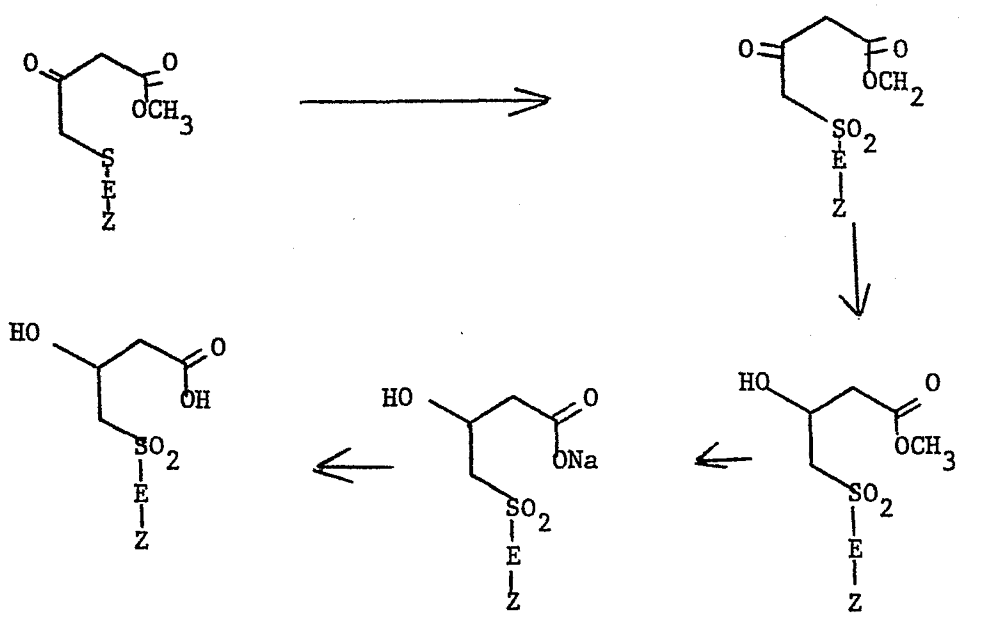

TABLE III

E=$-(CH_2)_2-$; $-CH_2-$; $-(CH_2)_3-$; $-CH_2CH_2CH_2-$

Z = 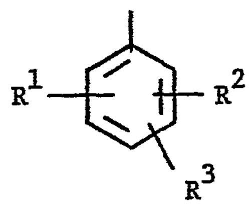

| $R^1$ | $R^2$ | $R^3$ |
|---|---|---|
| 6-(3'-methyl-4'-fluoro) | 2-$CH_3$ | 4-$CH_3$ |
| 6-(3',5'-dimethylphenyl | 2-$CH_3$ | 4-$CH_3$ |
| 6-(4'-fluorophenyl) | 2-Cl | 4-Cl |
| 6-(3',4'-dichlorophenyl) | 2-Cl | 4-Cl |
| 6-(3'-methyl-4'-fluorophenyl) | 2-Cl | 4-Cl |
| 6-(3',4'-dichlorophenyl) | 2-$CH_3$ | 4-$CH_3$ |
| 6-(3',4'-dichlorophenyl) | 2-$CH_3$ | 5-$CH_3$ |
| 6-(4'-fluorophenyl) | 2-$CH_3$ | 4-$CH_3$ |
| 6-(3'-methyl-4'-fluorophenyl) | 2-$CH_3$ | 4-Cl |
| 6-benzyloxy | 2-Cl | 4-Cl |
| 6-benzyloxy | 2-$CH_3$ | 4-$CH_3$ |
| 6-(4-fluorobenzyloxy | 2-$CH_3$ | 4-Cl |
| 6-n-pentyloxy- | 2-Cl | 4-Cl |
| 6-(2,4-dichlorobenzyloxy)- | 2-Cl | 4-Cl |
| 6-allyloxy- | 2-Cl | 4-Cl |
| 6-(3-phenyl-1-propoxy)- | 2-Cl | 4-Cl |
| 6-cinnamyloxy- | 2-Cl | 4-Cl |
| 6-(4-methylbenzyloxy)- | 2-Cl | 4-Cl |
| 6-(4-methoxybenzyloxy)- | 2-Cl | 4-Cl |
| 6-hexafluorobenzyloxy- | 2-Cl | 4-Cl |

TABLE III (cont'd)

| | | |
|---|---|---|
| H | 2-Cl | 4-Cl |
| H | 2-$CH_3$ | 4-$CH_3$ |
| 6-phenyl- | 2-Cl | 4-Cl |
| 6-phenoxy- | 2-Cl | 4-Cl |
| 6-(4-trifluoromethyl-benzyloxy)- | 2-Cl | 4-Cl |
| 6-(4-methoxyphenyl)- | 2-Cl | 4-Cl |
| 6-(4-methylphenyl)- | 2-Cl | 4-Cl |
| 6-hydroxy | 2-Cl | 4-Cl |
| 6-(4-fluorophenoxy)- | 2-Cl | 4-Cl |
| 6-(4-methylphenoxy)- | 2-Cl | 4-Cl |
| 6-(4-methoxyphenoxy)- | 2-Cl | 4-Cl |
| 6-cyclohexylmethoxy- | 2-Cl | 4-Cl |
| 6-benzyloxy- | 2-Cl | 4-Cl |
| 6-acetoxy- | 2-Cl | 4-Cl |
| 6-benzoyloxy- | 2-Cl | 4-Cl |
| 6-(4-trifluoromethylphenyl)- | 2-Cl | 4-Cl |
| 6-adanantylmethoxy- | 2-Cl | 4-Cl |
| 6-(2-chloro-1-ethoxy)- | 2-Cl | 4-Cl |
| 6-(4-acetylphenoxy)- | 2-Cl | 4-Cl |
| 6-(4-trifluoromethylphenoxy)- | 2-Cl | 4-Cl |
| 6-(4-acetylbenzyloxy)- | 2-Cl | 4-Cl |
| 6-(2-chloro-1-ethyl)- | 2-Cl | 4-Cl |
| 6-(4-acetylphenyl)- | 2-Cl | 4-Cl |

Z = 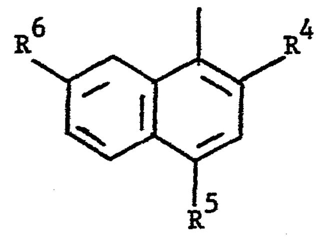

| $R^4$ | $R^5$ | $R^6$ |
|---|---|---|
| -Cl | -Cl | -Cl |
| $-CH_3$ | $-CH_3$ | $-CH_3$ |
| $-CH_3$ | $-CH_3$ | -F |
| $-CH_3$ | $-CH_3$ | -Cl |

EXAMPLE 9

7-[4'-Fluoro-3,3',5-trimethyl-(1,1'-biphenyl)-2-yl]-3-hydroxy-5,5-dioxo-5-thiaheptanoic acid

Step A: Preparation of Methyl 7-[4'-fluoro-3,3',5-trimethyl-(1,1'-biphenyl)-2-yl]-3-hydroxy-5,5-dioxo-5-thiaheptanoate

m-Chloroperbenzoic acid (0.86 g, 5.0 mmoles of 80-85% tech. reagent) was added to a solution of methyl 7-[4'-fluoro-3,3',5-trimethyl-(1,1'-biphenyl)-2-yl]-3-hydroxy-5-thiaheptanoate (0.68 g, 1.74 mmoles) in $CH_2Cl_2$ (6 mL) cooled in an ice-water bath. The bath was removed and the reaction was stirred at ambient temperature for 3 hours. The reaction was dissolved in ether (150 mL) and washed

successively with dilute sodium carbonate solution twice, then water, dried ($MgSO_4$), filtered and the solvent evaporated to leave 0.61 g of crude product containing a trace of sulfoxide. The impurity was removed by chromatography on a Still column, 40 x 150 mm, eluting with 29% acetone in hexane to give 0.41 g of pure methyl 7-[4'-fluoro-3,3',5-trimethyl-(1,1'-biphenyl)-2-yl]-3-hydroxy-5,5-dioxo-5-thiaheptanoate, m.p. 125-126°C.

Calc. for $C_{22}H_{27}FO_5S$: C, 62.54; H, 6.44;
Found: C, 62.94; H, 6.49.

Step B: Preparation of Sodium 7-[4'-fluoro-3,3',5-trimethyl-(1,1'-biphenyl)-2-yl]-3-hydroxy-5,5-dioxo-5-thiaheptanoate

The ester from Step A is converted to the sodium salt by the procedure described in Example 1, Step I.

Step C: Preparation of 7-[4'-fluoro-3,3',5-trimethyl-(1,1'-biphenyl)-2-yl]-3-hydroxy-5,5-dioxo-5-thiaheptanoic acid

The title free acid is prepared by the process described in Example 1, Step J.

EXAMPLE 10

Methyl 7-[4'-fluoro-3,3',5-trimethyl-(1,1'-biphenyl)-2-yl]-3-hydroxy-5-oxo-thiaheptanoate

Methyl 7-[4'-fluoro-3,3',5-trimethyl-(1,1'-biphenyl)-2-yl]-3-hydroxy-5-thiaheptanoate (5.15 g, 13.18 mmoles) was dissolved in methylene chloride (65 mL) and cooled to -78°C under nitrogen. m-Chloroperbenzoic acid (80--85% tech. grade, 2.73 g,

13.18 mmoles) was added slowly in divided portions over a period of 15 minutes. After the addition was complete, stirring at -78°C was continued for 30 minutes. Dimethyl sulfide (11 mL) was added and stirring continued at -78°C for 30 minutes. The reaction was then dissolved in ether and extracted 2 times with aqueous sodium bicarbonate solution, dried ($MgSO_4$), filtered and the solvent evaporated to give 5.12 g of a mixture of isomers. These isomers were separated by flash chromatography on a 100 mm diameter Still column containing 1 kg of silica gel 230-400 mesh eluting with 25% acetone in methylene chloride (v/v) to give isomer A, isomer B and a mixture. The mixture was rechromatographed on a 80 mm x 210 mm Still column containing silica gel 234-400 mesh, eluting with the same solvent to give a combined 2.02 g of isomer A, m.p. 97-100°C; 2.60 g of isomer B, m.p. 114-116°C and 0.28 g of a mixture of isomers A and B.

## EXAMPLE 11

**Methyl 7-[4'-fluoro-3,3',5-trimethyl-(1,1'-biphenyl)-2-yl]-3-hydroxy-5-thiaheptanoate**

The dried ether solution of 7-[4'-fluoro-3,3',5-trimethyl-(1,1'-biphenyl)-2-yl]-3-hydroxy-5-thiaheptanoic acid (Example 1, Step J) is treated with excess ethereal diazomethane until a yellow color persists. The reaction is allowed to stand for 15 minutes then nitrogen is bubbled through the solution until the excess diazomethane is gone (yellow color disappears). The ether is evaporated *in vacuo* to leave methyl 7-[4'-fluoro-3,3',5-trimethyl-(1,1'-biphenyl)-2-yl]-3-hydroxy-5-thiaheptanoate.

EXAMPLE 12

Ethyl 7-[4'-fluoro-3,3',5-trimethyl-(1,1'-biphenyl)-2-yl]-3-hydroxy-5-thiaheptanoate

Sodium 7-[4'-fluoro-3,3',5-trimethyl-(1,1'-biphenyl)-2-yl]-3-hydroxy-5-thiaheptanoate (from Example 1, Step I) (0.4 g, 1 mmole) is dissolved in DMF (2.0 mL). To this is added ethyl iodide (0.78 g, 5 mmoles). The reaction is stirred and heated at 50° for 24 hours or until tlc shows the starting sodium salt of the acid to be converted to the ester. The reaction mixture is partitioned between ether and water. The water is extracted 2 times with ether. The combined ether layers are washed with water 2 times, dried ($MgSO_4$) and the solvent and excess ethyl iodide are evaporated in vacuo to give ethyl 7-[4'-fluoro-3,3',5-trimethyl-(1,1'-biphenyl)-2-yl]-3-hydroxy-5-thiaheptanoate.

EXAMPLE 13

Ammonium 7-[4'-fluoro-3,3',5-trimethyl-(1,1'-biphenyl)-2-yl]-3-hydroxy-5-thiaheptanoate

The ethereal solution of 7-[4'-fluoro-3,3',5-trimethyl-(1,1'-biphenyl)-2-yl]-3-hydroxy-5-thiaheptanoic acid (from Example 1, Step J) is concentrated in vacuo to a volume of 0.5 mL. Dry gaseous ammonia is bubbled into the solution until the precipitation is complete. The reaction is stirred overnight and the precipitated ammonium 7-[4'-fluoro-3,3',5-trimethyl-(1,1'-biphenyl)-2-yl]-3-hydroxy-5-thiaheptanoate is collected.

EXAMPLE 14

2,3-Dihydroxypropyl 7-[4'-fluoro-3,3',5-trimethyl-(1,1'-biphenyl)-2-yl]-3-hydroxy-5-thiaheptanoate

Sodium 7-[4'-fluoro-3,3',5-trimethyl-(1,1'-biphenyl)-2-yl]-3-hydroxy-5-thiaheptanoate (0.41 g, 1 mmole) is dissolved in DMF (2 mL) and 1-iodo-2,3-dihydroxy propane (0.40 g, 2 mmole) is added. The reaction is heated at 80° overnight. After cooling to room temperature the reaction mixture is partitioned between ether and a small amount of water. The ether is dried ($MgSO_4$), filtered and the solvent is evaporated *in vacuo* to leave 2,3-dihydroxypropyl 7-[4'-fluoro-3,3',5-trimethyl-(1,1'-biphenyl)-2-yl]-3-hydroxy-5-thiaheptanoate which is purified by chromatography.

WHAT IS CLAIMED IS:

1. A compound of structural formula:

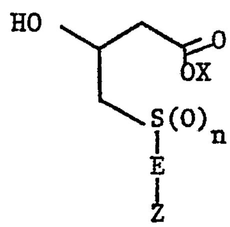

wherein Z is:

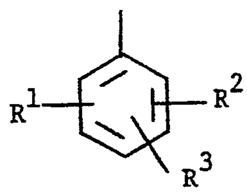

or

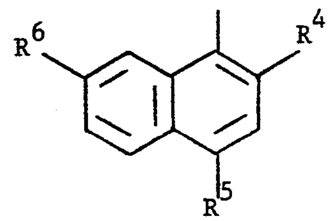

n is 0, 1 or 2;

E is $-CH_2-$, $-CH_2-CH_2-$, $-CH_2-CH_2-CH_2-$, $-CH=CH-CH_2-$; or $-CH_2-CH=CH-$;

$R_1$, $R_2$ and $R_3$ are each selected from:

1) hydrogen,
2) halogen, wherein halo is chloro, bromo or fluoro,
3) $C_{1-4}$alkyl,
4) $C_{1-4}$haloalkyl, wherein halo is chloro, bromo or fluoro,
5) phenyl,
6) phenyl substituted by one or more of:

a) halogen,
b) $C_{1-4}$ alkoxy,
c) $C_{2-8}$ alkanoyloxy,
d) $C_{1-4}$ alkyl, or
e) $C_{1-4}$ haloalkyl, and

7) $OR_7$ in which $R_7$ is
a) hydrogen,
b) $C_{2-8}$ alkanoyl,
c) benzoyl,
d) phenyl,
e) phenyl substituted with one or more of
  i) halogen,
  ii) $C_{1-4}$ alkoxy,
  iii) $C_{2-8}$ alkanoyloxy,
  iv) $C_{1-4}$ alkyl, or
  v) $C_{1-4}$ haloalkyl,
f) $C_{1-9}$ alkyl,
g) cinnamyl,
h) $C_{1-4}$ haloalkyl,
i) allyl,
j) cycloalkyl-$C_{1-3}$-alkyl,
k) adamantyl-$C_{1-3}$-alkyl,
l) phenyl $C_{1-3}$ alkyl,
m) phenyl $C_{1-3}$-alkyl, wherein the phenyl is substituted with one or more of:
  i) halogen,
  ii) $C_{1-4}$ alkoxy,
  iii) $C_{2-8}$ alkanoyloxy,
  iv) $C_{1-4}$ alkyl, or
  v) $C_{1-4}$ haloalkyl; and

$R^4$, $R^5$ and $R^6$ are each selected from:

1) hydrogen,
2) halo, wherein halo is chloro, bromo or fluoro, and
3) $C_{1-3}$ alkyl; and

X is
1) hydrogen,
2) $R^8$ wherein $R^8$ is $C_{1-3}$ alkyl,
3) M wherein M is a cation derived from an alkali metal, or is ammonium of formula $^+NR^9,R^{10},R^{11}, R^{12}$ wherein $R^9$, $R^{10}$, $R^{11}$ and $R^{12}$ are independently $C_{1-3}$alkyl, or two of $R^9$, $R^{10}$, $R^{11}$ and $R^{12}$ are joined together to form a 5 or 6-membered heterocycle with the nitrogen to which they are attached,
4) M' wherein M' is a cation derived from protonation of an amine of formula $NR^{13}R^{14}R^{15}$ wherein $R^{13}$,$R^{14}$ and $R^{15}$ are independently hydrogen or $C_{1-3}$ alkyl, or two of $R^{13}$, $R^{14}$ and $R^{15}$ are joined to form a 5 or 6-membered heterocycle with the nitrogen to which they are attached, or
5) $R^{16}$ wherein $R^{16}$ is
   a) $C_{1-5}$ alkyl, or
   b) 2,3-dihydroxypropyl.

2. The compound of Claim 1 wherein Z is:

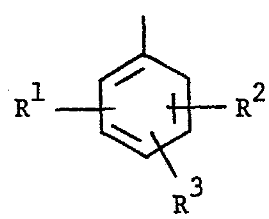

$n = 0, 1$ or $2$;

E is $-CH_2CH_2-$;

$R_1$, $R_2$ and $R_3$ are each selected from:

1) halogen,
2) $C_{1-4}$ alkyl,
3) $C_{1-4}$ haloalkyl,
4) phenyl substituted by one or more of
   a) halo,
   b) $C_{1-4}$ alkyl,
   c) $C_{1-4}$ alkoxy, and
5) $OR_7$ in which $R_7$ is
   a) phenyl,
   b) phenyl substituted by one or more of
      i) halogen, or
      ii) $C_{1-4}$ alkyl;
   c) phenyl-$C_{1-3}$ alkyl, or
   d) phenyl-$C_{1-3}$-alkyl substituted with one or more of
      i) halogen and
      ii) $C_{1-4}$ alkyl; and

X is
1) hydrogen,
2) $R^8$,
3) M, or
4) M'.

3. The compound of Claim 2 wherein Z is

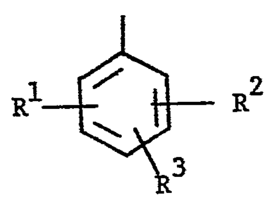

n is 0 or 1;

E is $-CH_2-CH_2-$;

$R_1$ is situated in the 6-position and is a substituted phenyl wherein there are 1 or 2 substituents and they are independently selected from chloro, fluoro, methyl and methoxy; and

$R_2$ and $R_3$ are halo or $C_{1-3}$ alkyl in the 2 and 4 positions; and

X is hydrogen, a sodium cation, or $C_{1-3}$ alkyl.

4. The compounds of Claim 3 of structural formula:

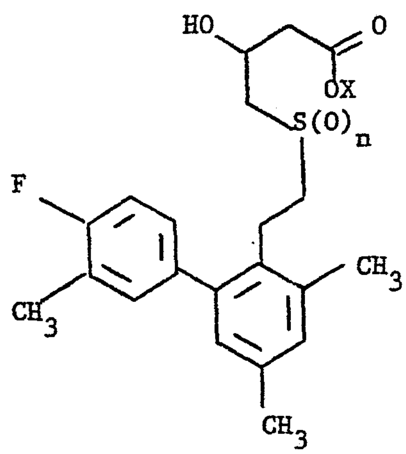

wherein X is hydrogen or a sodium cation and n=0 or 1.

5. An antihypercholesterolemic pharmaceutical formulation comprising a pharmaceutically acceptable carrier and an effective antihypercholesterolemic amount of a compound of Claim 1.

6. The formulation of Claim 5, wherein the compound is that of Claim 2.

7. The formulation of Claim 6 wherein the compound is that of Claim 3.

8. The formulation of Claim 7 wherein the compound is that of Claim 4.

9. A process for the preparation of a compound of structural formula:

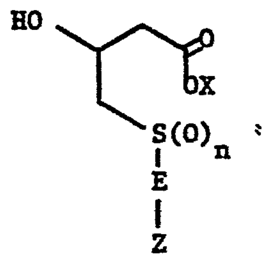

wherein Z is:

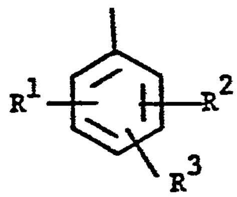

or

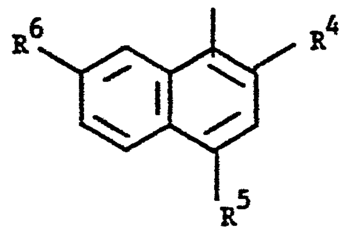

n is 0, 1 or 2;

E is $-CH_2-$, $-CH_2-CH_2-$, $-CH_2-CH_2-CH_2-$, $-CH=CH-CH_2-$, or $-CH_2-CH=CH-$;

$R_1$, $R_2$ and $R_3$ are each selected from :

1) hydrogen,
2) halogen, wherein halo is chloro, bromo or fluoro,
3) $C_{1-4}$alkyl,
4) $C_{1-4}$haloalkyl, wherein halo is chloro, bromo or fluoro,
5) phenyl,
6) phenyl substituted by one or more of:
   a) halogen,
   b) $C_{1-4}$ alkoxy,
   c) $C_{2-8}$ alkanoyloxy,
   d) $C_{1-4}$ alkyl, or
   e) $C_{1-4}$ haloalkyl, and

7) $OR_7$ in which $R_7$ is

a) hydrogen,

b) $C_{2-8}$ alkanoyl,

c) benzoyl,

d) phenyl,

e) phenyl substituted with one or more of

i) halogen,

ii) $C_{1-4}$ alkoxy,

iii) $C_{2-8}$ alkanoyloxy,

iv) $C_{1-4}$ alkyl, or

v) $C_{1-4}$ haloalkyl,

f) $C_{1-9}$ alkyl,

g) cinnamyl,

h) $C_{1-4}$ haloalkyl,

i) allyl,

j) cycloalkyl-$C_{1-3}$-alkyl,

k) adamantyl-$C_{1-3}$-alkyl,

l) phenyl $C_{1-3}$ alkyl,

m) phenyl $C_{1-3}$-alkyl, wherein the phenyl is substituted with one or more of:

i) halogen,

ii) $C_{1-4}$ alkoxy,

iii) $C_{2-8}$ alkanoyloxy,

iv) $C_{1-4}$ alkyl, or

v) $C_{1-4}$ haloalkyl; and

$R^4$, $R^5$ and $R^6$ are each selected from:

1) hydrogen,
2) halo, wherein halo is chloro, bromo or fluoro, and
3) $C_{1-3}$ alkyl; and

X is
1) hydrogen,
2) $R^8$ wherein $R^8$ is $C_{1-3}$ alkyl,
3) M wherein M is a cation derived from an alkali metal, or is ammonium of formula $\overset{+}{N}R^9,R^{10},R^{11}, R^{12}$ wherein $R^9$, $R^{10}$, $R^{11}$ and $R^{12}$ are independently $C_{1-3}$alkyl, or two of $R^9$, $R^{10}$, $R^{11}$ and $R^{12}$ are joined together to form a 5 or 6-membered heterocycle with the nitrogen to which they are attached,
4) M' wherein M' is a cation derived from protonation of an amine of formula $NR^{13}R^{14}R^{15}$ wherein $R^{13}$,$R^{14}$ and $R^{15}$ are independently hydrogen or $C_{1-3}$ alkyl, or two of $R^{13}$, $R^{14}$ and $R^{15}$ are joined to form a 5 or 6-membered heterocycle with the nitrogen to which they are attached, or
5) $R^{16}$ wherein $R^{16}$ is
   a) $C_{1-5}$ alkyl, or
   b) 2,3-dihydroxypropyl.

which comprises treating a compound of structural formula:

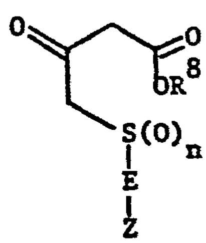

with sodium borohydride in a $C_{1-3}$ alkanol at -5 to +5°C, followed, if desired by:

a) if n=O, oxidation with a peracid to produce the compounds wherein n=1 and/or 2;

b) if n=1, separation of isomers;

c) saponification to produce the compound wherein X is M, followed by acidification to produce the compound wherein X is H, followed by treatment with a compound of formula $NR^9R^{10}R^{11}$ to produce the compound wherein X is M';

d) treatment of the compound wherein X is M with a $C_{1-5}$ alkyl halide or 2,3-dihydroxypropylhalide to produce the compound wherein X is $R^{16}$.